# EUROPEAN PATENT SPECIFICATION

(11) **EP 3 908 223 B1**
(45) Date of publication and mention of the grant of the patent: **15.07.2026**
(21) Application number: 20755276.1
(22) Date of filing: 11.02.2020
(51) Int. Cl.: A61B 34/30, A61M 25/01, A61M 25/08

(54) **ROBOTIC CATHETER SYSTEM ADAPTOR**
ADAPTER FÜR EIN ROBOTISCHES KATHETERSYSTEM
ADAPTATEUR POUR SYSTÈME DE CATHÉTER ROBOTIQUE

(30) Priority: 11.02.2019 US 201962803858 P
(43) Date of publication of application: 17.11.2021
(73) Proprietor: Siemens Healthineers Endovascular Robotics, Inc., Newton, MA 02466 (US)
(72) Inventor: FALB, Peter, Hingham, Massachusetts 02043 (US); GREGORY, Paul, Watertown, Massachusetts 02472 (US); COPE, Jason, Natick, Massachusetts 01760 (US); BLACKER, Steven J., Framingham, Massachusetts 01701 (US); BERGMAN, Per, West Roxbury, Massachusetts 02132 (US)
(74) Representative: HKW Intellectual Property PartG mbB
(86) International application number: PCT/US2020/017638
(87) International publication number: WO 2020/167749

(56) References cited:
- WO-A1-2005/072807
- WO-A1-2010/031581
- WO-A1-2010/107916
- WO-A1-2014/039838
- WO-A1-2015/066055
- CN-B- 107 307 909
- US-A1- 2010 130 987
- US-A1- 2014 171 919
- US-A1- 2015 173 782
- US-A1- 2016 271 368
- US-A1- 2016 271 368
- US-A1- 2017 000 979
- US-A1- 2017 049 995
- US-B1- 8 206 359
- VALWORX: "Valworx 5881 High Temperature Valve Mounting Kit", 19 January 2017 (2017-01-19), XP093311281, Retrieved from the Internet <URL:https://www.youtube.com/watch?v=A5sUeoeetrI>

## Description

### CROSS-REFERENCE TO RELATED PATENT APPLICATIONS

This application claims the benefit of US Provisional Application No 62/803858 entitled Robotic Catheter System Adapter (Atty Dkt. C130-352) filed on February 1] 2019.

### BACKGROUND OF THE INVENTION

The present invention relates generally to the field of catheter procedure systems and, in particular, a system for navigating a device (e.g., an elongated medical device) through a path (e.g., a vessel).

Catheters (and other elongated medical devices) may be used for many minimally-invasive medical procedures for the diagnosis and treatment of diseases of various vascular systems, including neurovascular interventional (NVI) also known as neurointerventional or neuroendovascular surgery, percutaneous coronary intervention (PCI) and peripheral vascular intervention (PVI). These procedures typically involve navigating a guidewire through the vasculature, and via the guidewire advancing a working catheter to deliver therapy. The catheterization procedure starts by gaining access into the appropriate vessel, such as an artery or vein, with a sheath or guide catheter using standard percutaneous techniques. The sheath or guide catheter is then advanced over a guidewire and/or diagnostic guidewire to the primary location such as an internal carotid artery for NVI, a coronary ostium for PCI or a superficial femoral artery for PVI. A guidewire and/or microcatheter suitable for the vasculature is then navigated through the sheath or guide catheter to a target location in the vasculature. In certain situations, such as in tortuous anatomy, a support catheter or microcatheter is inserted over the guidewire to assist in navigating the guidewire. The physician or operator may use an imaging system (e.g., fluoroscope) to obtain a cine with a contrast injection and select a fixed frame for use as a roadmap to navigate the guidewire or catheter to the target location, for example a lesion. Contrast-enhanced images are also obtained while the physician delivers the guidewire or catheter device so that the physician can verify that the device is moving along the correct path to the target location. While observing the anatomy using fluoroscopy, the physician manipulates the proximal end of the guidewire or catheter to direct the distal tip into the appropriate vessels toward the lesion and avoid advancing into side branches.

Robotic catheter procedure systems have been developed that may be used to aid a physician in performing catheterization procedures such as, for example, NVI, PCI and PVI. Examples of neurovascular intervention (NVI) catheter procedures include coil embolization of aneurysms, liquid embolization of arteriovenous malformations and mechanical thrombectomy of large vessel occlusions in the setting of acute ischemic stroke. In NVI, the physician uses a robotic system to gain lesion access by manipulating a neurovascular guidewire and microcatheter to deliver the therapy to restore normal blood flow. The access is enabled by the sheath or guide catheter but may also require an intermediate catheter for more distal territory or to provide adequate support for the microcatheter and guidewire. The distal tip of a guidewire is navigated into, or past, the lesion depending on the type of lesion and treatment. For treating aneurysms, the microcatheter is advanced into the lesion and the guidewire is removed and several coils are deployed into the aneurysm through the microcatheter and used to embolize the aneurysm. For treating arteriovenous malformations, a liquid embolic is injected into the malformation via a microcatheter. Mechanical thrombectomy to treat vessel occlusions can be achieved either through aspiration or use of a stent retriever. Aspiration is either done directly through the microcatheter, or with a larger bore aspiration catheter. Once the aspiration catheter is at the lesion, negative pressure is applied to remove the clot through the catheter. Alternatively, the clot can be removed by deploying a stent retriever through the microcatheter. Once the clot has integrated into the stent retriever, the clot is retrieved by retracting the stent retriever and microcatheter into the guide catheter.

In PCI, the physician uses a robotic system to gain lesion access by manipulating a coronary guidewire to deliver the therapy and restore normal blood flow. The access is enabled by seating a guide catheter in a coronary ostium. The distal tip of the guidewire is navigated past the lesion and, for complex anatomies, a microcatheter may be used to provide adequate support for the guidewire. The blood flow is restored by delivering and deploying a stent or balloon at the lesion. The lesion may need preparation prior to stenting, by either delivering a balloon for pre-dilation of the lesion, or by performing atherectomy using, for example, a laser or rotational atherectomy catheter and a balloon over the guidewire. Diagnostic imaging and physiological measurements may be performed to determine appropriate therapy by using imaging catheters or FFR measurements.

In PVI, the physician uses a robotic system to deliver the therapy and restore blood flow with techniques similar to NVI and PCI. The distal tip of the guidewire is navigated past the lesion and a microcatheter may be used to provide adequate support for the guidewire for complex anatomies. The blood flow is restored by delivering and deploying a stent or balloon to the lesion. As with PCI, lesion preparation and diagnostic imaging may be used as well. Relevant prior art can be found in US 2014/171919 A1, US 2016/271368 A1, WO 2010/031581 A1, WO 2005/072807 A1 and WO 2015/066055 A1.

### SUMMARY

The invention is defined by the appended claims. Methods mentioned herein do not form part of the claimed invention.

In one embodiment an adaptor for a robotic catheter system includes a body defining an opening configured to encompass an outer rotatable portion of hemostasis valve, the outer rotatable portion being rotatable within the opening. A distal end connector is configured to engage a portion of the hemostasis valve. A proximal end connector configured to connect to an elongated medical device support sheath or track.

In one embodiment a clip for a robotic catheter system includes a body releasably engaging an elongated medical device support track movable relative to a robotic drive. The body covers a slit in the support track. The body has a proximal end including an automatic detachment release configured to disengage the body from the support track when the proximal end of the clip contacts the robotic drive.

A robotic catheter system includes a catheter mechanism movable relative to a base. A controller robotically moves the catheter mechanism relative to the base between at least a first predetermined position and a second predetermined position.

A method for selecting a loading position of a robotic catheter system includes providing a catheter mechanism robotically movable relative to a base, and providing a user input selecting between a first predetermined position and a second predetermined position.

### BRIEF DESCRIPTION OF THE DRAWINGS

This application will become more fully understood from the following detailed description, taken in conjunction with the accompanying figures, wherein like reference numerals refer to like elements in which:
Figure 1 is an isometric view of a robotic catheter system.
Figure 2 is top isometric view of a front portion of the robotic catheter system of Figure 1 with an exploded view of a guide catheter and Y-connector.
Figure 3 is a side front view of the front portion of the robotic catheter system of Figure 2 with the guide catheter positioned within a Y-connector support in a raised position.
Figure 4 is an isometric view of the portion of the system of Figure 2 with the Y-connector and support in a lowered position with the Y-connector support cover in the raised position.
Figure 5 is a top plan view of the front portion of the robotic catheter system of Figure 2 with the guide catheter in the engaged position.
Figure 6A is an isometric view of the robotic catheter system with the sheath clip in an install position.
Figures 6B is an isometric view of the robotic catheter system with the sheath clip in an engaged position.
Figure 7 is an exploded view of the arcuate portion of the rigid guide and front of the robotic catheter system.
Figure 8 is a close up of the sheath clip, flexible track and rigid support of Figure 7.
Figure 9 is an exploded view of the sheath clip and distal end of the rigid guide.
Figure 10 is an isometric view of the front portion of the robotic catheter system with the flexible track in an extended position.
Figure 11 is cross-sectional view of the front portion of the robotic catheter system taken generally along line 11-11 of Figure 5 showing an extension member protruding into a slit of the flexible track.
Figure 12 is a cross-sectional view of the front portion of the robotic catheter system taken generally along lines 12-12 of figure 6A with the sheath clip in an in-load position.
Figure 13 is a cross-sectional view of the front portion of the robotic catheter system taken generally along lines 13-13 of figure 6B with the sheath clip in the operational position.
Figure 14 is a top plan view of the robotic catheter system with the flexible track in the fully retracted position.
Figure 15 is a top plan view of the robotic catheter system with the flexible track in an extended position.
Figure 16 is a top plan view of the robotic catheter system with the robotic drive in a first position.
Figure 17 is a top plan view of the robotic catheter system with the robotic drive in a second extended position.
Figure 18 is a rear isometric view of the robotic catheter system with a linear drive.
Figure 19 is an exploded rear isometric view of the robotic catheter system with the cassette in a pre-assembly position relative to the robotic drive base.
Figure 20 is a rear isometric view of the robotic catheter system with the cassette secured to the robotic drive base with the locking track clamp in the disengaged position.
Figure 21 is a close up view of the locking track clamp taken generally along lines 21 - 21 of figure 20.
Figure 22 is a close-up isometric view of the locking track clamp in an engaged position.
Figure 23 is a cross-sectional view of the locking track clamp in an engaged position and unlocked.
Figure 24 is an exploded view of a portion of the locking track clamp.
Figure 25A is a cross-sectional view of the locking track clamp in an unlocked position.
Figure 26A is a cross-sectional view of the locking track clamp in an unlocked position.

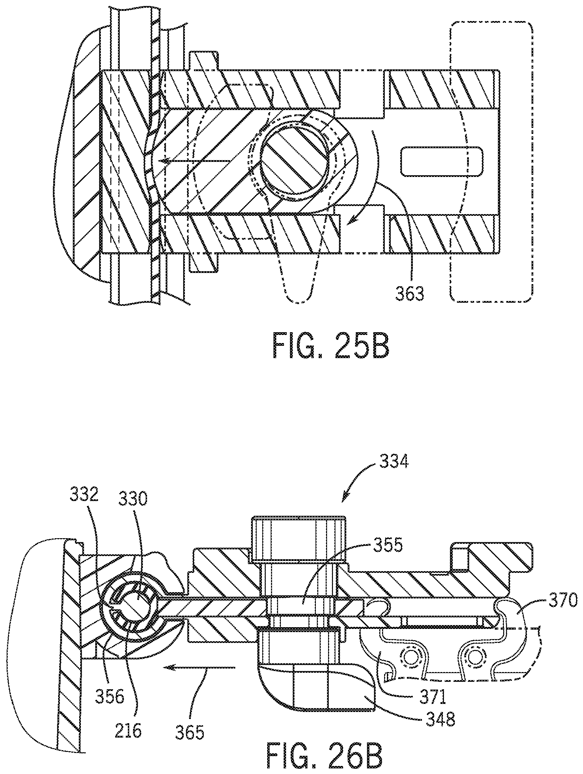

Figure 25B is a cross-sectional view of the locking track clamp in a locked position.
Figure 26B is a cross-sectional view of the locking track clamp in the locked position.

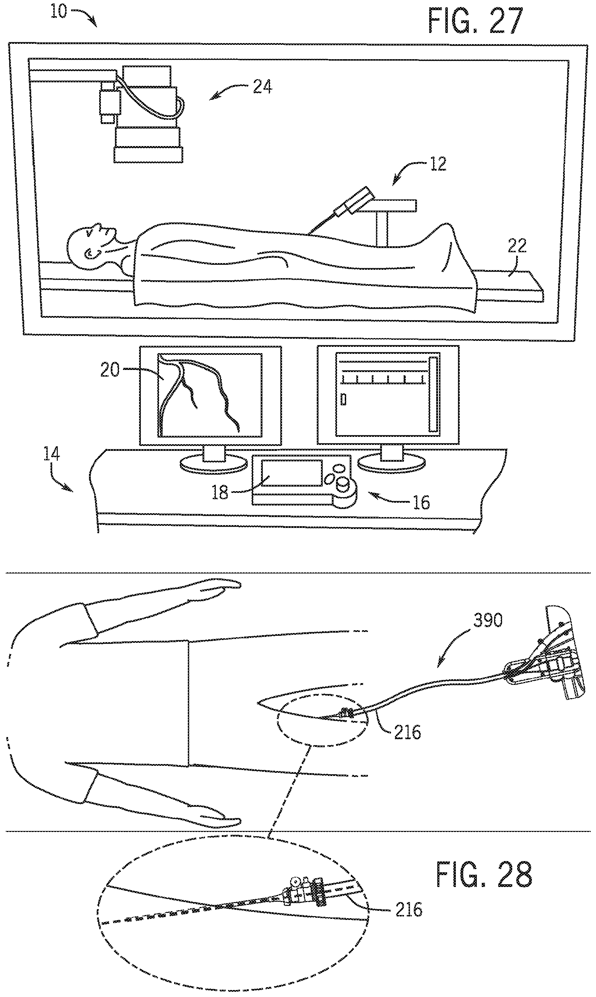

Figure 27 is a schematic view of the robotic catheter system with a remote control station.
Figure 28 is illustration of robotic catheter system with the guide catheter engaged with a patient.

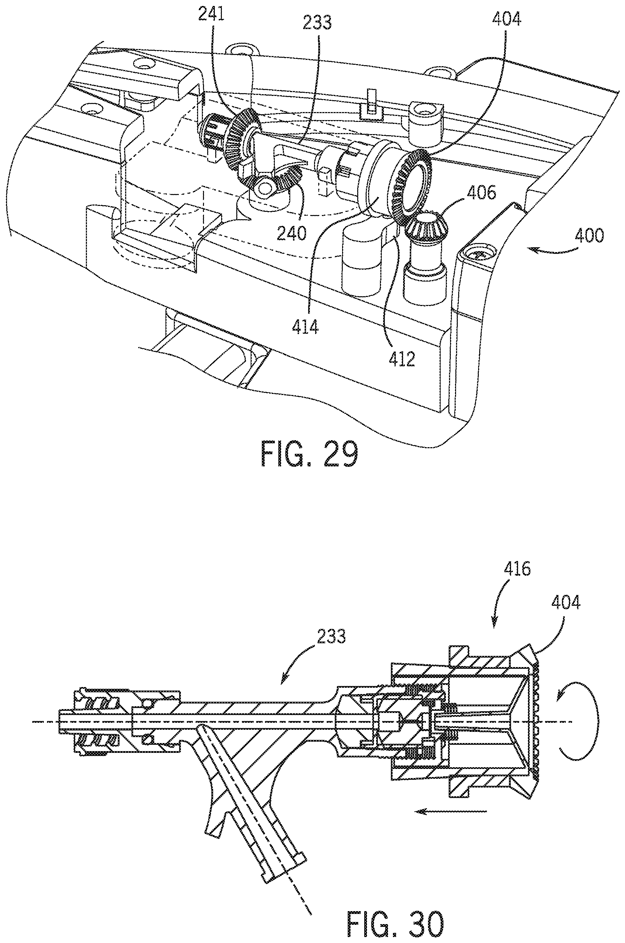

Figure 29 is a view of a hemostasis valve control mechanism.
Figure 30 is a cross-sectional view of the hemostasis valve illustrating the opening and closing the back portion of the hemostasis valve.

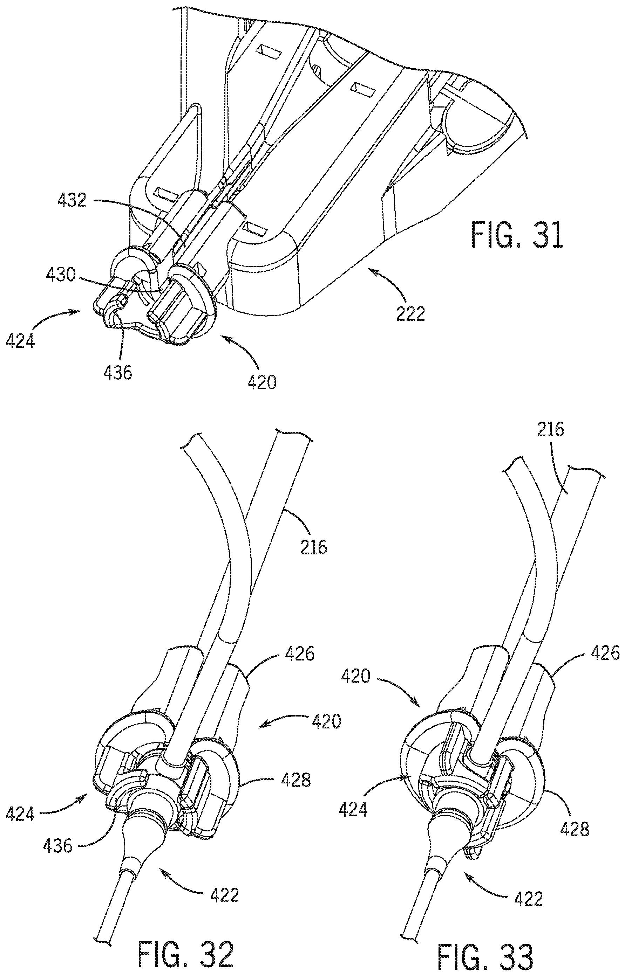

Figure 31 is an isometric view of a sheath clip.
Figure 32 is an isometric view of the sheath clip of Figure 31 with an introducer.
Figure 33 is an isometric view of the sheath clip of Figure 31 with an introducer connected to the sheath clip.

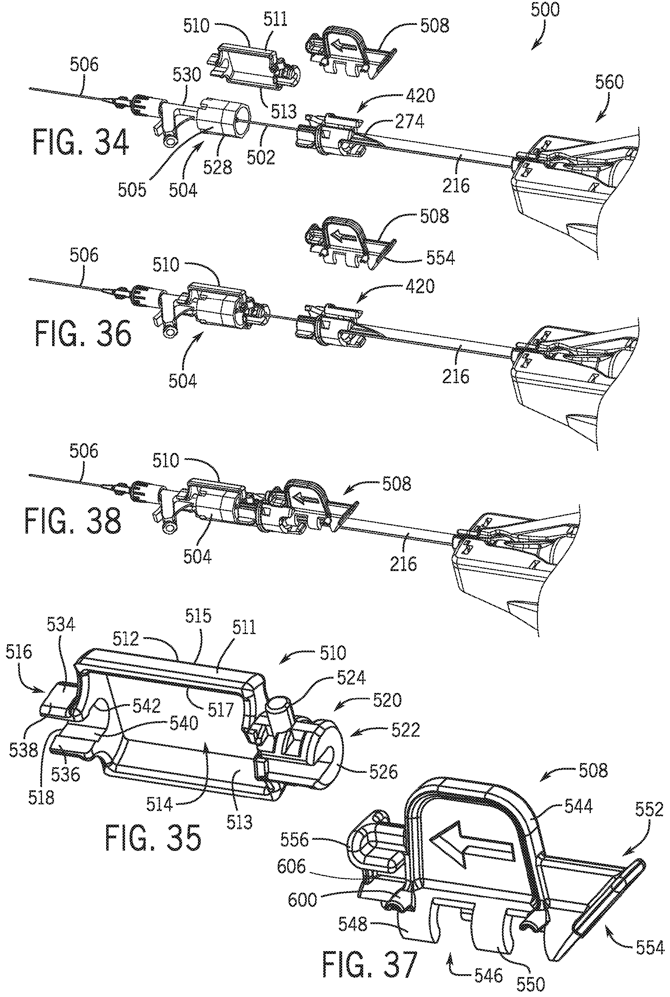

FIG 34 is an exploded view of a robotic catheter system.
FIG 35, is an isometric view of an adaptor.
FIG 36 is the robotic catheter system of FIG 34 with the adaptor secured to a hemostasis valve.
FIG 37 is an isometric view of a clip.
FIG 38 is the robotic catheter system of FIG 34 with the flexible track coupler secured to the adaptor and the clip secured to the flexible track.

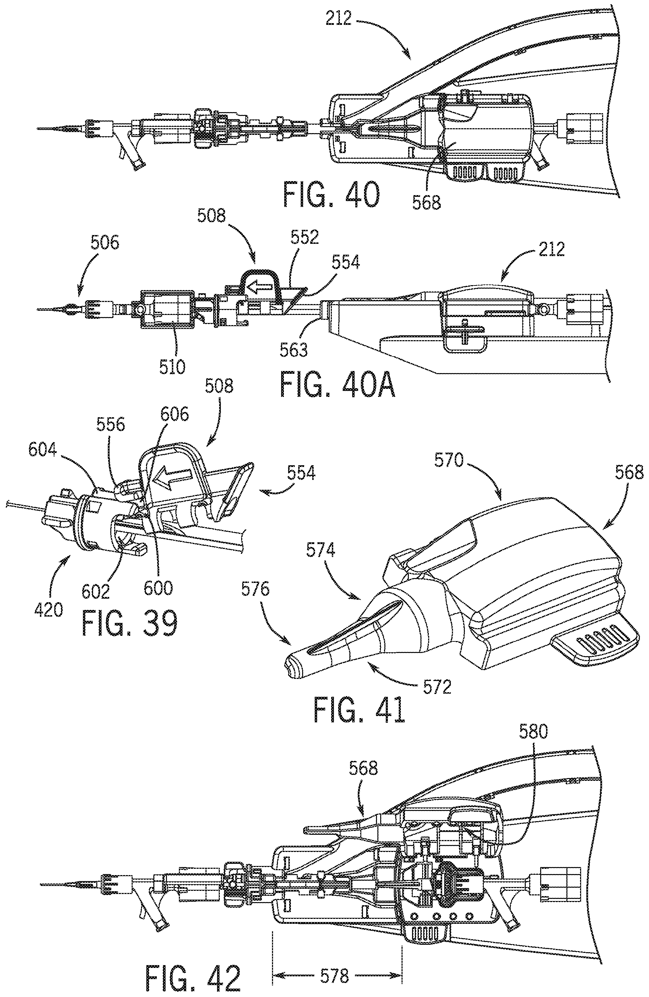

FIG 39 is an isometric view of the clip released from the flexible track.
FIG 40 is a top plan view of the catheter system with a cover in the closed position, the catheter system being a partial view of the entire system.
FIG 40A is a side plan view of the catheter system with a cover in the closed position.
FIG 41 is a perspective view of the cover and perspective view of the secured to the flexible track.
FIG 42 is a top plan view of the catheter system with the cover in the open position the catheter system being a partial view of the entire system.

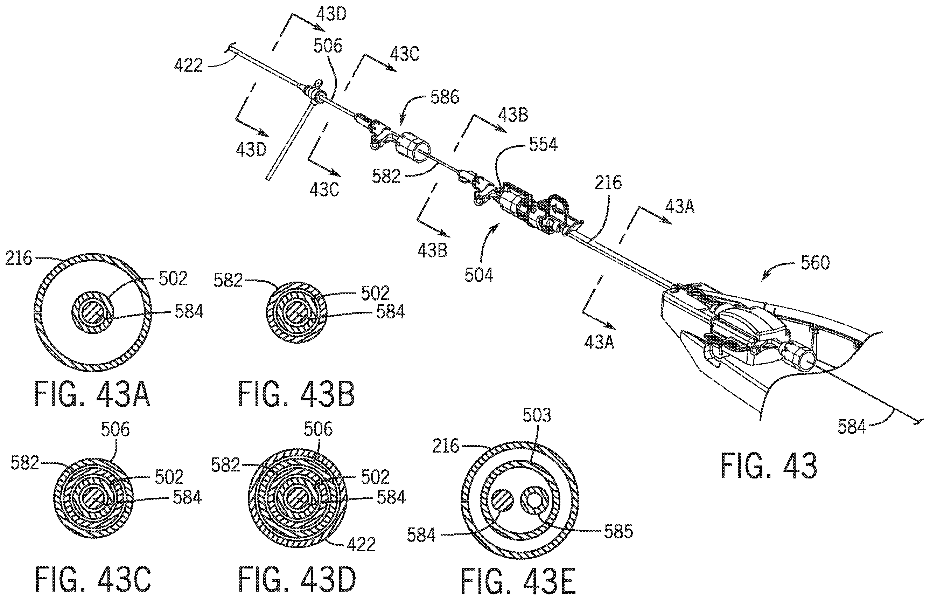

FIG 43 is an isometric view of a catheter system with an intermediate sheath and a distal sheath.
FIG 43A is a schematic local cross section taken generally along lines 43A-43A of figure 43.
FIG 43B is a schematic local cross section taken generally along lines 43B-43B of figure 43.
FIG 43C is a schematic local cross section taken generally along lines 43C-43C of figure 43.
FIG 43D is a schematic local cross section taken generally along lines 43D-43D of figure 43.
FIG 43E is a schematic local cross section taken generally along lines 43A-43A with a catheter within the hollow lumen of the controlled catheter.

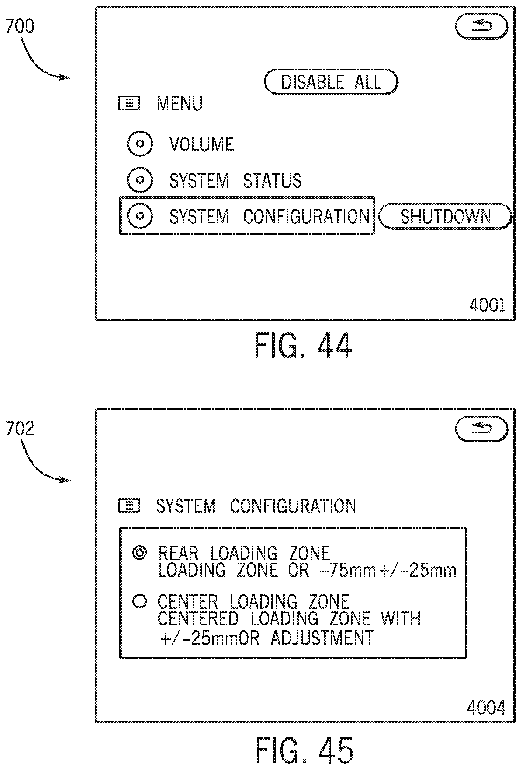

FIG 44 is a display having menu option including system configuration.
FIG 45 is a display having a menu option between a read and forward loading configuration.

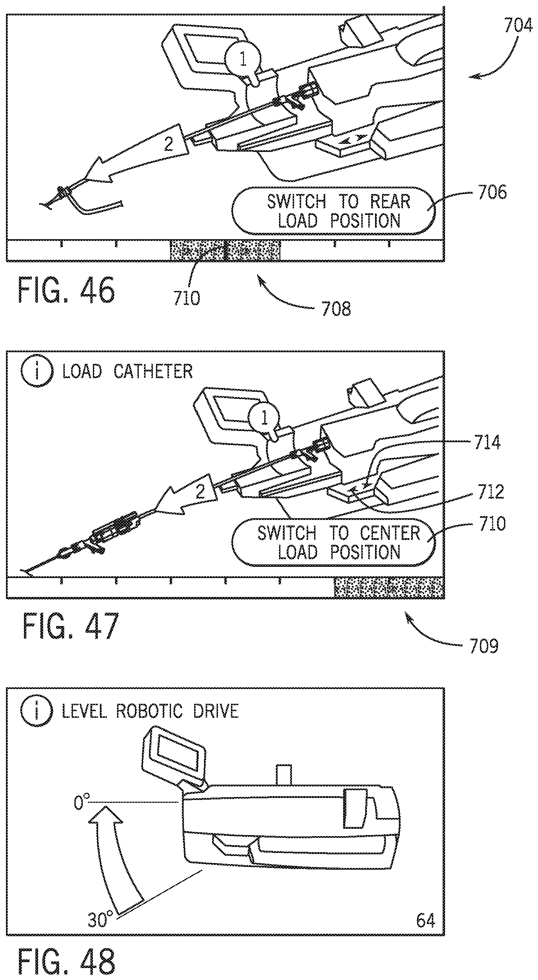

FIG 46 is a display of a robotic mechanism in a center loading configuration.
FIG 47 is a display of a robotic mechanism in a rear loading configuration.
FIG 48 is a display of the level of a robotic mechanism relative to a base.

### DETAILED DESCRIPTION OF THE PREFERRED EMBODIMENTS

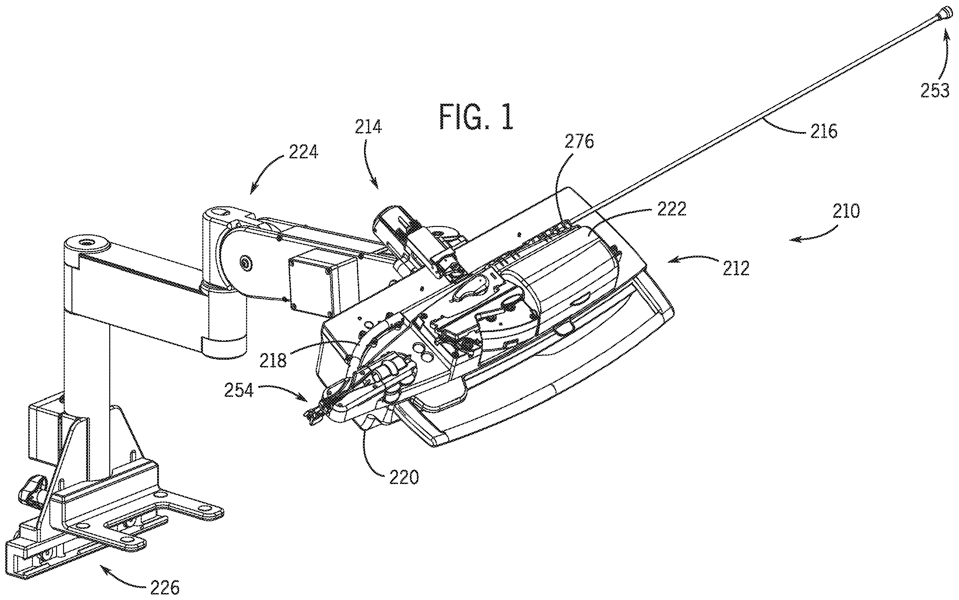

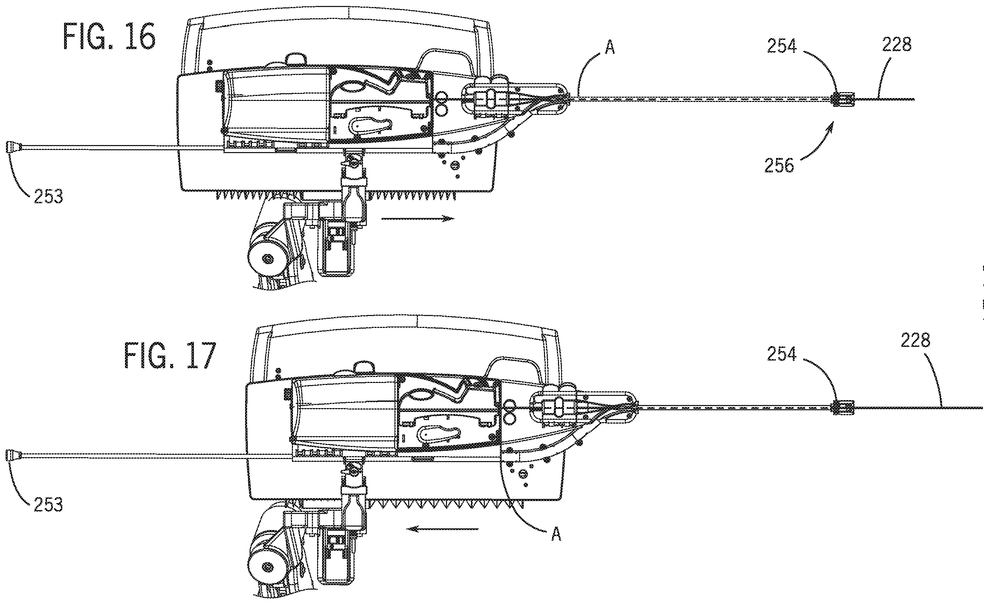

Referring to Figure 1 a robotic catheter system 210 includes a robotic mechanism 212 robotically moving an elongated medical device. The robotic mechanism 212 is movable relative to a base 214. A flexible track 216 is movable along a rigid guide track 218 having a non-linear portion. Referring to Figure 16 flexible track 216 includes a proximal end 253 and a distal end 254.

As described in more detail herein, flexible track 216 supports an elongated medical device such as a guide catheter so that the guide catheter can be advanced into the patient without buckling.

As used herein the direction distal is the direction toward the patient and the direction proximal is the direction away from the patient. The term up and upper refers to the general direction away from the direction of gravity and the term bottom, lower and down refers to the general direction of gravity. The term front refers to the side of the robotic mechanism that faces a user and away from the articulating arm. The term rear refers to the side of the robotic mechanism that is closest to the articulating arm. The term inwardly refers to the inner portion of a feature. The term outwardly refers to the outward portion of a feature.

Robotic mechanism 212 includes a robotic drive base 220 movable relative to base 214 and a cassette 222 that is operatively secured to robotic drive base 220. In one embodiment cassette 222 includes structure that defines support rigid guide 218. In one embodiment base 214 alone or in combination with cassette 222 defines rigid guide 218.

In one embodiment base 214 is secured to an articulating arm 224 that allows a user to position robotic mechanism 212 proximate a patient. In one embodiment base 214 is the distal portion of the articulating arm 224. Articulating arm 224 is secured to a patient bed by a rail clamp or a bed clamp 226. In this manner base 214 is secured to a patient bed. By manipulation of articulated arm 224 the base 214 is placed in a fixed location relative to a patient that lies upon the patient bed. The arms of articulated arm 224 can be fixed once the desired location of robotic mechanism 212 is set relative to the patient.

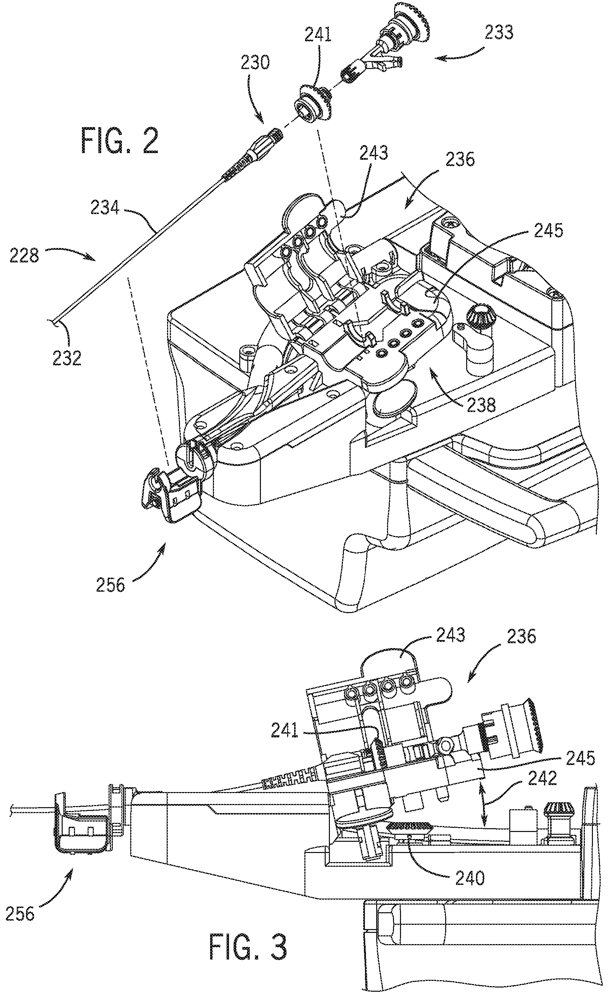

Referring to Figure 2 an elongated medical device such as a guide catheter 228 is operatively secured to robotic mechanism 212 through cassette 222. Guide catheter 228 includes a proximal end 230, an opposing distal end 232, and an intermediate portion 234 extending between the proximal end 230 and distal end 232. In one embodiment proximal end 230 of guide catheter 228 is operatively secured to a Y-connector 233 and Y-connector engagement mechanism 236. In one embodiment Y-connector 233 is a hemostasis valve that is secured to cassette 222 by a Y-connector engagement mechanism 236 including a Y-connector base 238 that is part of cassette 222 and an enclosure member 244 including a lid 243 and a support member 245. Y-connector base 238 includes a guide catheter drive mechanism 240 located in the cassette 222 which in turn is operatively connected to robotic base 220. Guide catheter drive mechanism 240 includes a drive mechanism that operatively engages and rotates guide catheter 228 along its longitudinal axis correction about its longitudinal axis based on commands provided by a remote control center.

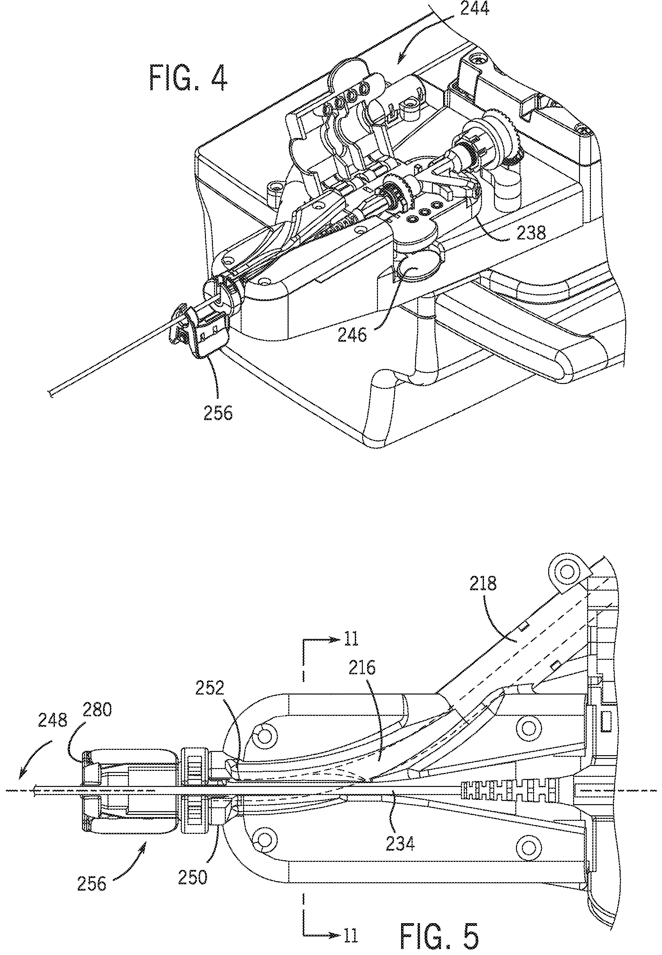

Referring to Figure 3, Y connector enclosure 244 pivots to a raised install position to provide easy installation of guide catheter 228 and Y-connector 233. Referring to Figure 4, the Y-connector enclosure 244 pivots along vector 242 from the raised position to an in-use operational lower position. In one embodiment guide catheter drive mechanism 240 interacts with a gear 241 on a rotating luer lock connector secured to proximal end 230 of guide catheter 228 to robotically rotate guide catheter 228 about its longitudinal axis. The operation of a Y-connector holder and drive mechanism 236 to robotically rotate guide catheter 228 about its longitudinal axis is described in published US Patent Application No. US 2014/0171863 A1 entitled Hemostasis Valve for Guide Catheter Control The robotic control of the Y-connector hemostasis valve 233 will be discussed in further detail below.

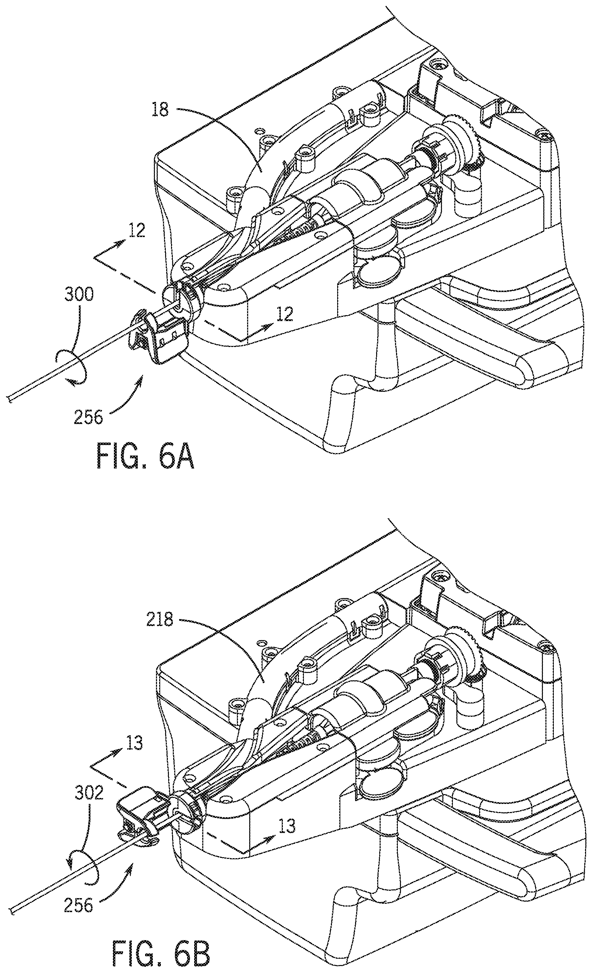

Referring to Figure 4 and Figure 6, Y connector holder 238 includes a cover 244 which pivots from an open position to a closed position. Y connector holder 238 is releasably engaged to a portion of cassette 222 by a release button 246. Movement of lever 246 allows Y connector holder 238 to be pivoted from the operational lower position to the raised position to load guide catheter 228 and Y-connector 233.

Referring to Figure 5 the relationship between guide catheter 228, rigid guide 218, and flexible track 216 will be described. Guide catheter 228 maintains a linear position along its longitudinal axis 248 within cassette 222 and for at least a certain distance distal cassette 222. In one embodiment longitudinal axis 248 corresponds to the longitudinal axis of cassette 222.

During a medical procedure such as a percutaneous coronary intervention (PCI) guide catheter 228 is used to guide other elongated medical devices such as a guide wire and balloon stent catheter into a patient to conduct an exploratory diagnosis or to treat a stenosis within a patient's vasculature system. In one such procedure the distal end 232 of the guide catheter 228 is seated within the ostium of a patient's heart. Robotic mechanism 212 drives a guide wire and/or a working catheter such as a balloon stent catheter in and out of a patient. The guide wire and working catheter are driven in within the guide catheter 228 between the distal end of the robotic mechanism 212 and the patient. In one embodiment longitudinal axis 248 is the axis about which cassette 222 causes rotation of a guide wire and the axis along which cassette 222 drives the guide wire along its longitudinal axis and drives a working catheter such as a balloon stent catheter along its longitudinal axis. In one embodiment the robotic drive system is of the type described in US Patent No. 7,887,549 entitled Catheter System Robotic drive systems include a first actuator driven by a motor operatively coupled to a device drive that provides linear and/or rotary motion to an elongated medical device such as a catheter and a guidewire and other devices known in the percutaneous device art. The device drive may use rollers, pads or other known engagement mechanisms to impart linear and/or rotary motion to the elongated medical devices. In one embodiment robotic drive systems include a second actuator driven by a motor operatively coupled to a device drive that provides linear motion to a catheter.

Referring to Figures 5, 7 and 9 a collar 250 is formed at the distal end of rigid guide 218. Collar 250 includes a vertically extending opening 278 through which guide catheter 228 is loaded into flexible track 216.

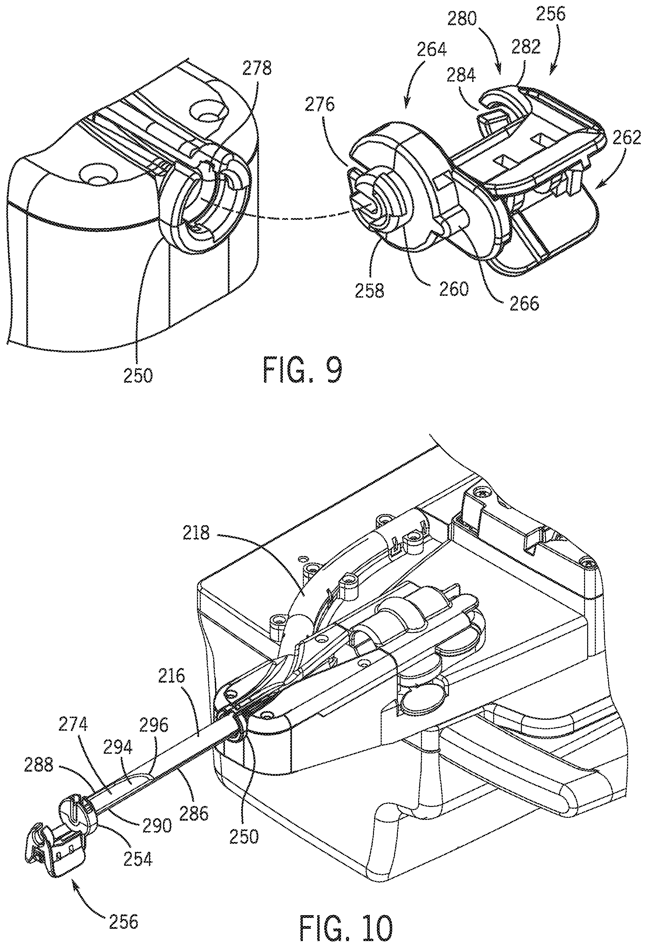

The terminal end 254 of flexible track 216 is secured to a sheath clip 256 which is releasably connected to cassette 222. Flexible track 246 includes a collar 250 secured to a terminal distal end 252. Referring to Figure 9 in one embodiment a sheath clip 256 includes a proximal end 258 including an attachment portion 260. The distal end 254 of flexible track 216 is secured to attachment portion 260. Sheath clip 256 includes a grasping portion 262 that allows a user to manipulate sheath clip 256 and flexible track 216. Intermediate the grip portion 262 and the flexible track attachment portion 260 is a collar engagement portion 264. Collar engagement portion 264 and includes a guiding locating member 266 configured to position sheath clip 256 within collar 250.

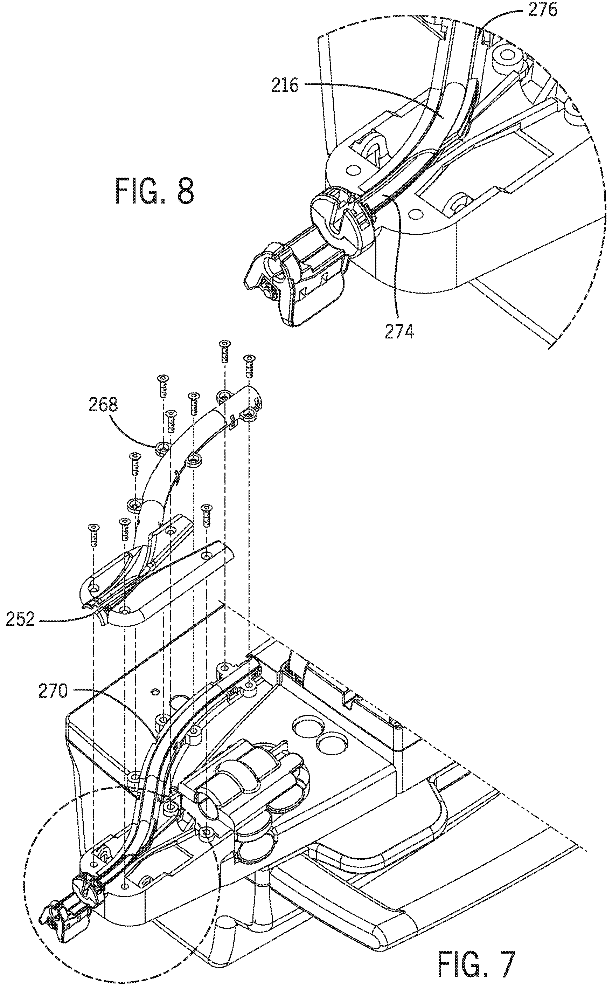

Referring to Figure 7 rigid guide 218 includes a top member 268 and a bottom channel member 270. Top member 268 and bottom channel member 270 when secured together with a plurality of fasteners or other fastening mechanism forms a interior channel 272 through which flexible track 216 moves relative to rigid guide 218.

Referring to Figure 8 flexible track 216 includes an opening 274 located adjacent to the terminal end 254 of flexible track 216 a predetermined distance toward proximal end of flexible track 216. When distal end 254 of flexible track 216 is positioned adjacent collar 250 opening 274 extends from collar 250 toward Y-connector holder a distance sufficient such that opening 274 extends from collar 250 to the area in which rigid guide 218 begins an arcuate path away from longitudinal axis 248. In one embodiment arcuate path forms an s-curve having at least one point of inflection along the arcuate path. As discussed below opening 274 provides a path for guide catheter 228 to be placed into the hollow cavity of flexible track directly from a position above longitudinal axis. In this manner guide catheter 228 may be placed within flexible track 216 proximate opening 274 while guide catheter 228 is linear. Stated another way in one embodiment guide catheter 228 is in a straight line when the guide catheter 228 is inserted through opening 274. In one embodiment opening 274 extends 90 degrees about the opening of the terminal end 254 of flexible track 216. Opening 274 tapers to a slit 286 that extends substantially the entire length of a flexible track or tube 216. In one embodiment slit 286 extends from opening 274 a distance sufficient to allow guide catheter 228 to enter and exit an interior portion of flexible track 216 throughout the entire intended operation of robotic catheter system. Opening 274 is defined by a pair of substantially parallel cut lines 288, 290 in the outer surface of flexible track 216. Opening 274 is further defined by a tapered region 294 with an arcuate line 296 extending from cut line 288 toward slit 286. In one embodiment flexible track 216 has sufficient rigidity to maintain slit 286 in the open position, that is the two portions of the outer surface of flexible track 216 that define slit 286 remain separated during movement of the flexible track 216 as described herein and do not collapse onto one another such that no opening is present. In one embodiment slit 286 collapses during certain portions of flexible track 216 as it moves through certain sections of rigid guide 218. In one embodiment slit 286 collapses that is the two edges that define the slit are in contact with one another except in the area in which guide catheter 228 enters and exits flexible track 218. The edges defining the slit are forced apart by extension member 298 in the region where the longitudinal axis 248 is coincident with the portion of rigid guide that begins the non-linear arcuate portion.

Referring to Figure 1 the distal end of flexible track 216 is fed into the channel of rigid guide 218 through its proximal opening 276. Rigid guide 218 includes a linear portion beginning at proximal opening 276 and a non-linear portion defined by cover 268 and base 270. In one embodiment the non-linear portion is an arcuate portion having at least one point of inflection. Flexible track 216 is initially positioned within rigid guide 218 by feeding distal end 254 of flexible track 218 into proximal opening 276 of rigid guide 218 until the distal end 254 of flexible track 216 extends beyond collar 250 of rigid guide 218. The distal end 254 of flexible track 216 is operatively connected to member 258 of sheath clip 256. Sheath clip 258 is positioned within collar 250 such that member 266 is positioned within a corresponding mating groove in collar 250. Sheath clip 256 is positioned in a first load position with channel opening 276 of sheath clip 258 aligned with opening 278 of collar 250.

Flexible track 216 is rotated by a technician or operator within rigid guide 218 such that opening 274 faces in an upwardly direction. Stated another way opening 274 of flexible track 216 is secured to sheath clip 256 in a manner such that when she clip 256 is engaged with collar 250 opening 276 of sheath clip 256 is aligned with opening 278 of collar 250 which is also aligned with opening 274 of flexible track 216.

Referring to figure 10 flexible track 216 is secured to sheath clip 256 with a portion of flexible track 216 extending beyond collar 250 in the distal position. The extension of the distal end 254 of flexible track 216 allows for easy insertion of flexible track 216 to sheath clip 256. Since flexible track 216 is formed of a flexible material having a modulus of elasticity that is less than the modulus of elasticity of the rigid guide material, flexible track 216 moves along the curved non-lineal portion of channel defined by rigid guide 218. Note that the modulus of elasticity of flexible track 216 is below a value in which flexible track 216 will fracture or break by movement along the non-linear portion of rigid guide 218. In one embodiment flexible track 216 is formed of a polytetrafluoroethylene PTFE material. Sheath clip 256 along with the terminal end 254 of flexible track 216 is moved adjacent to collar 250. Sheath clip 256 along with flexible track 216 is rotated to such that the opening 276 of sheath clip 256 is alignment with opening 278 of collar 250 defining the guide catheter installation position. As discussed below in one embodiment a sheath clip 420 is configured to be received within cassette 222 in the proper install orientation.

Referring to Figure 5 guide catheter 228 is positioned within opening 274 of flexible track 216 through opening 278 of collar 250 and through opening 276 of sheath clip 256. Referring to Figure 5 and Figure 9 the distal end 280 of sheath clip 256 includes a collar 282 having an opening 284. Guide catheter 228 in the installation position extends into flexible track 216 through opening 274, through opening 278 of collar 250 and through openings 276, 284 of sheath clip 256. In this installation position guide catheter 228 maintains a straight and linear orientation along its longitudinal axis 248 from Y- connector holder 236 through the distal end of sheath clip 256.

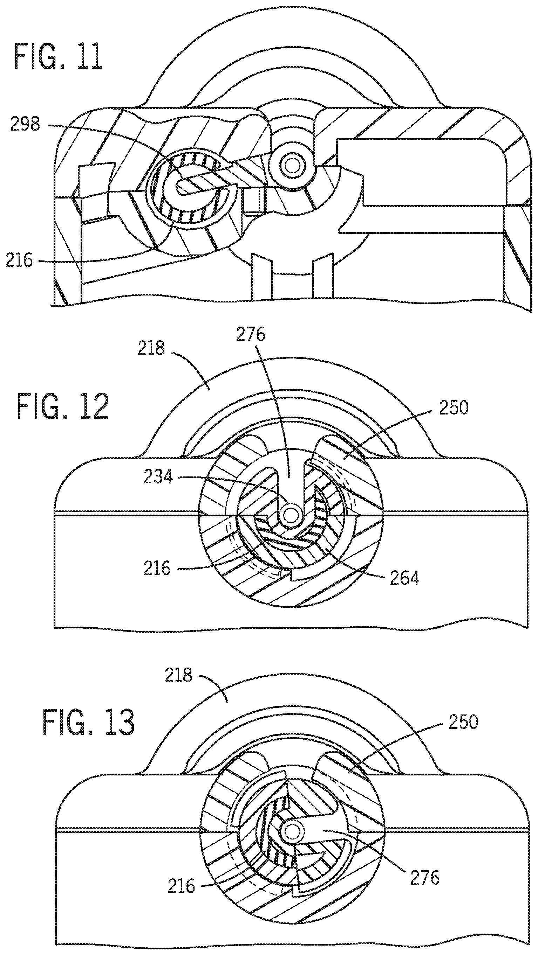

Referring to Figure 11, rigid guide 218 includes an extension member 298 that extends into the channel defined by the outer walls of rigid guide 218. Extension member 298 is received into the inner channel of flexible track 218 through slit 286. Extension member 298 is positioned proximate the distal end 300 of the arcuate portion of rigid guide 218. Extension member 298 has a thickness that is equal to or greater than the opening defined by slit 286 to ensure that the edges of flexible track 216 that define the slit remains separated so that guide catheter 228 can extend into the channel portion of flexible track 216 through the slit. In one embodiment the thickness of extension member 298 is greater than the opening defined by the slit and the diameter of the guide catheter 228. In this manner the opening defined by the slit 286 is increased at and closely adjacent to the extension member allowing for insertion and removal a portion of guide catheter 228. In one embodiment the opening defined by the slit is less than the diameter of the guide catheter 228 which assists in maintaining the distal portion of the guide catheter within the channel of the flexible track 216 during operation of the robotic catheter system.

Referring to Figure 6A, and Figure 12, sheath clip 256 is placed in an installation position with opening 276 in the upward direction. Stated another way opening 276 is formed by a channel in sheath clip 256 defining an opening that is accessed from the upward direction. This orientation allows guide catheter 228 to be positioned within the channel of sheath clip 256 and opening 274 of flexible track 216 in the same orientation that guide catheter is secured to cassette 222. In this orientation guide catheter 228 can be placed into the channel of flexible track 216 through openings 276 and 284 of sheath clip 256 and through opening 274 of flexible track 216.

Referring to Figure 6B and Figure 13, in one embodiment sheath clip 256 is rotated about longitudinal axis 248 until opening 276 extends 90 degrees from the vertical orientation sown in figure 12. In this manner guide catheter 228 is assisted in remaining within the channel of flexible track 216. As sheath clip 256 is rotated 90 degrees, extension member 298 acts to widen the opening defined by slit 286 immediately adjacent the longitudinal axis 248. In this manner guide catheter 228 can enter and exit flexible track 216 without interference from the edges of the flexible track that defines slit 286. In one embodiment described below a sheath clip 420 does not need to be rotated but simply pulled distally away from cassette 222.

In one embodiment sheath clip 256 is rotated in a first direction 90 degrees illustrated in Figure 6B, while in another embodiment sheath clip 256 is rotated 90 degrees in a direction opposite to the direction. It is also contemplated that sheath clip 256 may be rotated less than or greater than 90 degrees. In one embodiment described below sheath clip 420 does not need to be rotated.

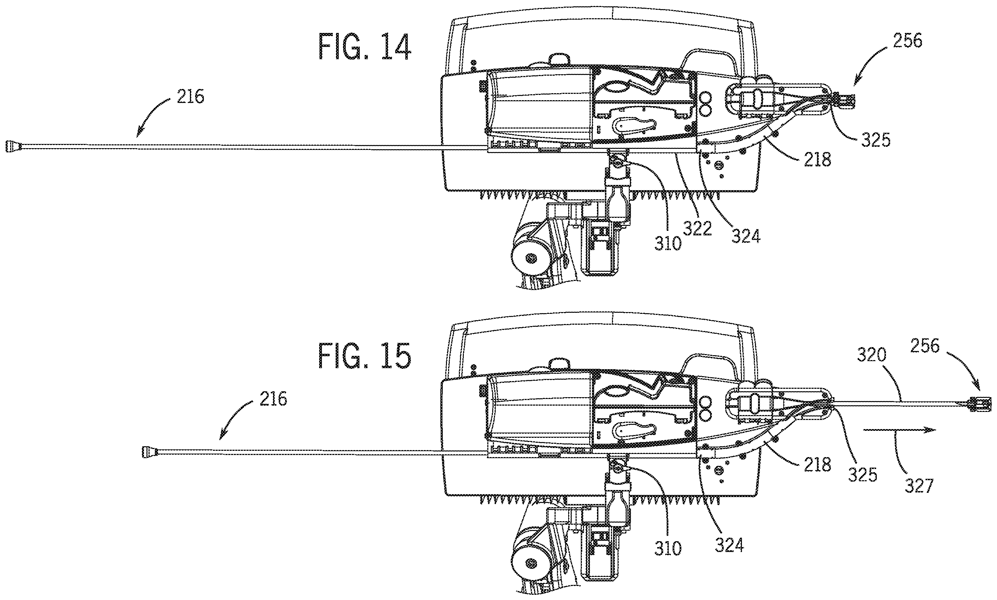

Referring to Figure 14 and Figure 15 in one embodiment once sheath clip 256 has been rotated to the operation position shown in Figure 13, the sheath clip is pulled by a user away from cassette 222 in a direction along longitudinal axis 248 until the distal end 280 sheath clip 256 is proximate the patient. In one embodiment an introducer is secured to distal end 280 of sheath clip 256. The introducer is a device that is secured to a patient to positively position the introducer to the patient to allow insertion and removal of elongated medical devices such as the guide catheter, guide wire and or working catheter into the patient with minimal tissue damage to the patient. Once the operator has pulled the sheath clip and accompanying flexible track toward the patient such that the introducer is proximate the patient, the flexible track is locked in position by a locking clamp 310.

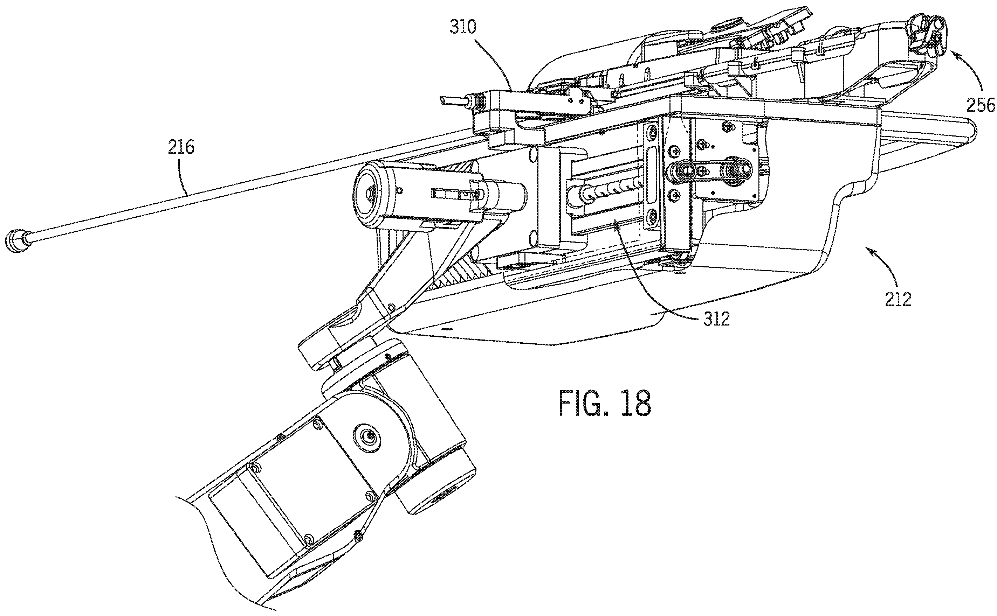

Locking clamp 310 secures flexible track 216 to base 214 such that a portion of flexible track 216 is in a fixed position relative to the patient bed and the patient to the extent the patient lies still on the patient bed. Referring to Figure 18, a linear drive mechanism 312 includes a linear slide that is robotically controlled by a user through a remote control station. Catheter drive mechanism drive 312 drives robotic mechanism 212 along longitudinal axis 248. Since rigid guide 218 is fixed relative to robotic mechanism 212, flexible track 216 moves relative to the rigid guide 218 as the robotic mechanism 212 moves along the longitudinal axis 248.

Referring to Figures 14, 15, 16 and 17 the operation and movement of flexible track 216 relative to rigid guide 218 will be described. Referring to figure 14 flexible track 216 is shown in the installation first position in which guide catheter 228 is positioned within sheath clip 256 and flexible track opening 274 as described above. Referring to Figure 15, once sheath clip 256 has been released from the cassette 222 as described above the sheath clip 256 and distal end of the flexible track are pulled by a user away from cassette 222 such that the distal end of the sheath clip 256 is proximate the entry point of the patient in which a percutaneous intervention will occur. As described below in further detail locking clamp 310 operatively clamps a portion of flexible track 216 that flexible track 216 fixed relative to base 214.

Referring to figures 14 and 15 the portion of flexible track 216 that is positioned within arcuate portion of rigid guide 218 is pulled out of the distal end of rigid guide 218 in a direction generally along longitudinal axis 248. Similarly a portion 322 of flexible track 216 that was external to and not located within the arcuate portion of rigid guide 218 is pulled into the arcuate portion of rigid guide 218 and depending on how far the terminal end of the flexible track is pulled toward the patient portion 322 of flexible track 216 will enter the arcuate portion of rigid guide 218 and may extend therefrom. Stated another way flexible track 216 includes three general regions that change with the operation of the guide catheter system. First a proximal region that includes the flexible track portion from the proximal end 253 to the opening 324 of the arcuate portion of rigid guide 218. Flexible track 216 includes a second portion located between the proximal end 324 of the arcuate portion of rigid guide 218 and the distal end 325 of the arcuate portion of rigid guide proximate collar 250. Flexible track includes a third region that extends from collar 250 of rigid guide 218 in a direction defined by a vector generally along longitudinal axis 248, where the vector has a beginning at Y-connector and extends in a direction toward collar 250.

The first region and second region of flexible track 216 as described above is offset from and not in line with longitudinal axis 248. The third portion of flexible track 216 is generally coaxial with longitudinal axis 248 as flexible track 216 exits collar 250 of rigid guide 218.

During one type of intervention procedure, guide catheter 228 is inserted into a patient's femoral artery through an introducer and positioned proximate the coronary ostium of a patient's heart. An operator may wish to relocate the distal end of the guide catheter robotically. Referring to Figure 16 and Figure 17 the control of the distal end of guide catheter 228 will be described. Referring to Figure 16 guide catheter 228 has a distal portion which extends beyond the distal end of sheath clip 256 in order to extend beyond the terminal end of guide catheter 228 in a direction away from the terminal end of sheath clip. As noted above the distal end of guide catheter 228 may be placed proximate the ostium of a patient. The robotic control of the distal end of guide catheter 228 is accomplished by movement of robotic drive mechanism 212 relative to base 214 by linear drive 312. Guide catheter is located within the channel of the flexible track from cassette 222 until the sheath clip 256. Since flexible track 216 is secured relative to base 214 the second portion of flexible track 216 as described above will move from within the arcuate portion of rigid guide 218 to a position offset from longitudinal axis 248. Similarly a third portion of flexible track 216 that extended distally beyond collar 250 will be retracted and moved into the arcuate portion of rigid guide 218 and in doing so is moved away from and offset from longitudinal axis 248.

If during a PCI procedure guide catheter begins to slip out of the ostium it is possible to extend the distal end of guide catheter 228 back into the patient's ostium by robotically moving the robotic drive 212 toward the patient. In doing so the distal end of guide catheter 228 is moved toward the patient reinserting or seating the distal end of the guide catheter into the patient's ostium as one example. As the robotic drive mechanism 212 is moved along longitudinal axis 248 flexible track 216 is moved relative to rigid guide 218. In actual operation a portion of flexible track 216 is fixed in space relative to base 214 at locking clamp 310. However, the portion of flexible track 216 that is located within the arcuate section of rigid guide 216 is moved toward and away from longitudinal axis 248 depending on the direction that the robotic drive mechanism 212 is moving. Guide catheter 228 moves into or out of the section of the flexible track 216 that is moving in and out of the arcuate portion of rigid guide 218. In this manner the portion of the guide catheter 228 between cassette 222 and the sheath clip is always located within the channel of flexible track 216. In this manner guide catheter 228 may be manipulated within flexible track 216 without buckling or causing other non-desirable movement during a percutaneous intervention procedure.

Referring to Figure 16 and Figure 17 the movement of flexible track 216 with respect to rigid guide 218 will be described as it relates to single section A on flexible track 216. In one example section A on flexible track 216 is located distal collar 250 of rigid guide 218.

When an operator determines to insert guide catheter 228 further into or toward a patient in a direction away from collar 250 an input device is manipulated by the user at a remote-control station that drives robotic drive 212 distally along longitudinal axis 248 by activating linear drive 312. The proximal end of guide catheter 228 is longitudinally fixed in cassette 222 by clamp 310 so that as the robotic drive 312 including cassette 222 is moved relative to base 214 by linear drive 312, in a direction toward the patient guide catheter 228 moves distally along longitudinal axis 248. As a result, the distal end of guide catheter 228 moves toward and/or into the patient.

As the robotic drive mechanism is moved along longitudinal axis 248 section A of flexible track 216 moves into rigid guide 218 through collar 250 and is moved along the arcuate portion of rigid guide 218 until section A of the flexible track 216 is adjacent the proximate opening of rigid guide 218. In this manner distal end of flexible track remains in a constant position but section A of flexible track 216 is moved out of or offset to the longitudinal axis 248. As section A moves from a point proximate the collar 250 into the arcuate channel defined by the rigid guide 218 the guide catheter 228 enters the channel or hollow lumen of the flexible track 216 through the slit adjacent in the engagement zone proximal to collar 250. In this manner flexible track 216 provides continual support and guidance for the guide catheter 228 between the collar 250 and patient as the distal end of guide catheter 228 is moved toward and away from the patient.

Similarly, if the operator desires to retract the distal end of the guide catheter 228 from within the patient, the user provides a command to the linear drive through the remote control station to move robotic drive mechanism 212 in a direction away from the patient. In this way section A of the flexible track 216 would enter the proximal end of the arcuate portion of the rigid guide and be guided within the channel of the rigid guide 218 until section A exits the distal end of the rigid guide 218. The guide catheter 228 would enter the slit at section A or stated another way a portion of the guide catheter 228 would enter the flexible track 216 via the portion of the slit that is located within the concentric circle taken at section A of the flexible track. Note that although sections of flexible track are positioned in different regions of the rigid guide as the robotic mechanism in moved toward and away from the patient the proximal end and the distal end of the flexible track remain in a fixed location as the robotic mechanism is moved along the longitudinal axis.

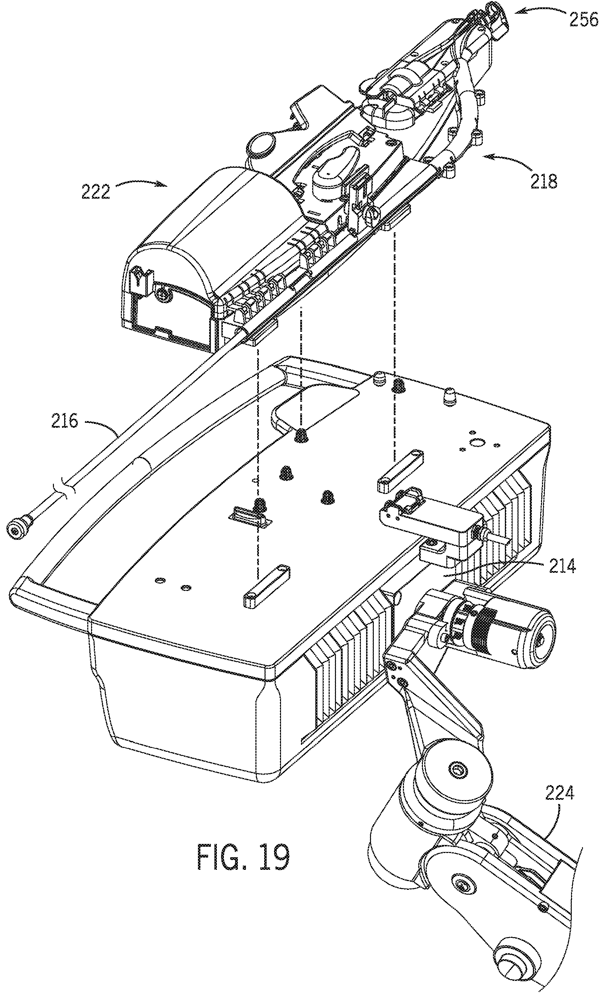

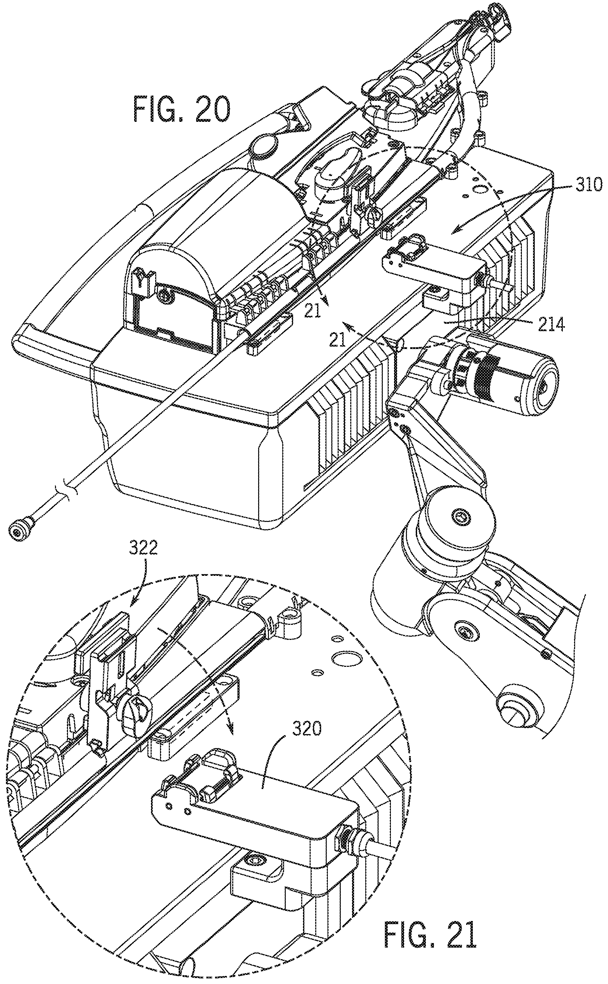

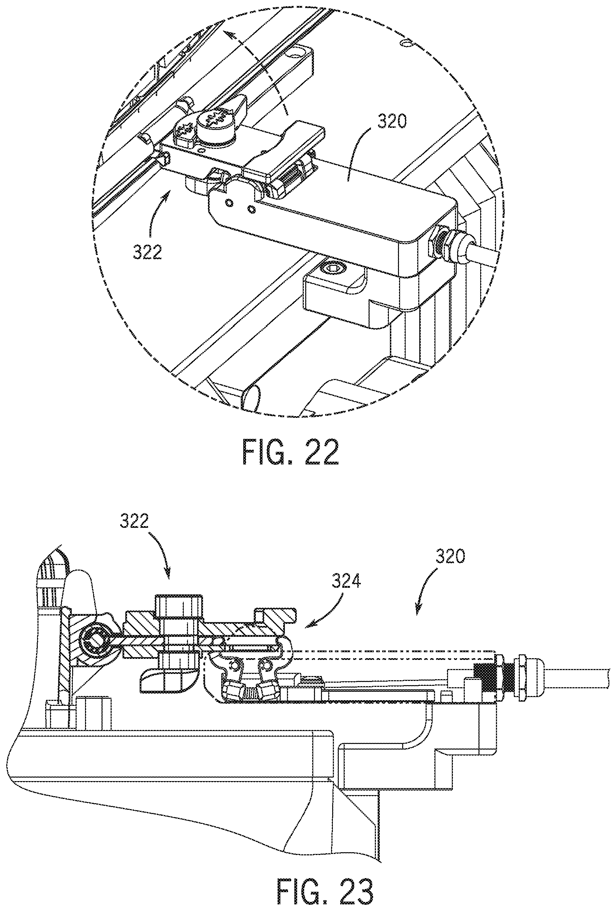

Referring to Figures 19-26 locking clamp 310 includes a base portion 320 operatively connected to base 214 and a clamp portion 322 that is coupled to base portion 320 via an engagement mechanism 324. Engagement mechanism 324 includes a pair of clasps 370, 371 on base portion 320 that engage a portion 357 via two indentations or grooves 360 and 362 on clamp portion 322. Clamp portion 322 includes a body 326 having a rigid guide connector 328 that is pivotally received in an opening in the rigid guide. Connector 328 includes a cylindrical member 356 that is received within the opening in rigid guide 218. Referring to Figure 21 Clamp portion 322 is in a raised position that can be used to ship the cassette separately from robotic drive base 220 without clamp portion 322 extending outwardly or rearwardly from cassette 222. Clamp portion 322 pivots about the longitudinal axis of rigid guide 218 proximate the opening in rigid guide 218 to an outwardly orientation to be coupled to base portion 320.

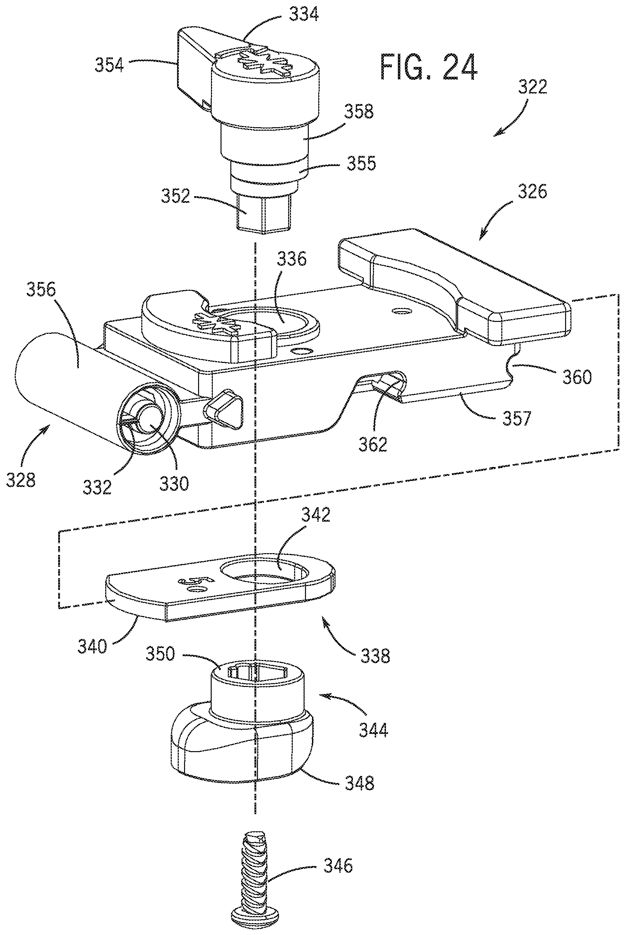

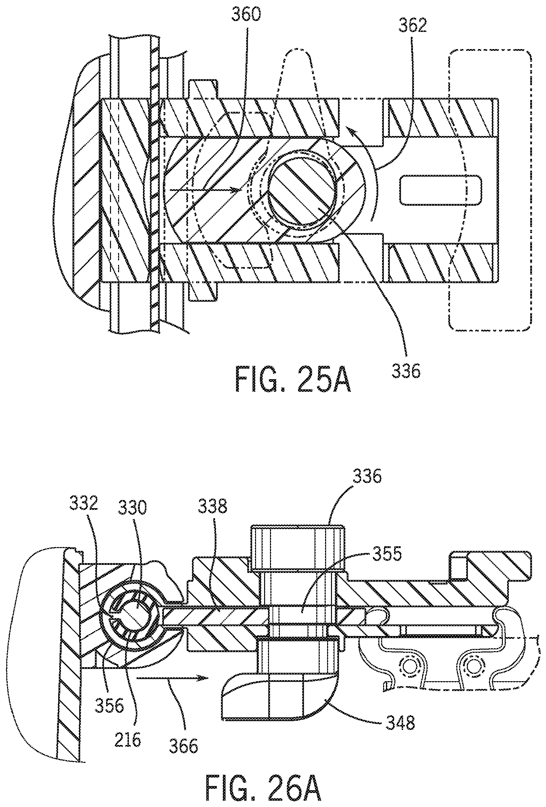

Referring to Figure 24, cylindrical member 356 defines a channel extending therethrough through which flexible track 216 extends. Extending inwardly into the channel from the cylindrical member 356 is a flat support 332. An inner cylindrical guide member 330 extends from flat support 332 such that cylindrical guide member is coaxial with the cylindrical member 356. Flexible track 216 is threaded through a proximal opening in rigid guide 218 and is passed over inner cylindrical guide member 330 such that the slit in flexible track 216 passes over flat support 332. In this manner flexible track 216 is positioned between inner cylindrical guide member 330 and cylindrical member 356. Referring to Figure 26A cylindrical member 356 includes a longitudinal opening through which a cam member 338 extends from body 326 toward the region defined between the inner cylindrical guide member 330 and the cylindrical member 356. As described below cam member 338 acts to lock flexible track 216 against inner cylindrical guide member 330.

Cam lock portion 322 includes a handle member 334 having a handle portion 354 and bearing surface 358 and a cam portion 355. Handle member 334 includes a keyed post 352 that is connected to a bottom key receptacle 344 through keyed opening 350. A fastener secures handle 334 to bottom key receptacle 344. Body 326 includes an opening 336 through which bearing 358 and cam 355 extend. Cam plate 338 includes an aperture 342 having an inner surface. Cam plate 338 includes a locking surface 340. In operation cam plate 342 is positioned within a slot in body 326 such that opening 342 is aligned with opening 336.

Referring to Figures 25A, 26A in the unlocked position lock surface 340 does not abut flexible track 216. Referring to Figures 25B and 26B bearing member 358 cooperates with the wall of opening 336 to centrally locate handle 334 within opening 336. Cam member 355 is positioned within opening 342 of cam plate 338 such that when handle 334 is rotated locking surface 340 is moved toward and away from rigid guide 218 to operatively y lock and unlock flexible track 216 relative to lock 310 and thereby to base 214.

Referring to Figure 29 and 30 Y-connector 233 is a hemostasis valve 402 that includes a valve body with a first leg having a proximal port, a distal port and a lumen extending between the proximal port and the distal port. At least one valve is located in the lumen adjacent the proximal port to permit an interventional device to be passed therethrough. The valve body includes a second leg extending at an angle relative to the first leg and in fluid communication with the first leg. A rotating male luer lock connector is rotatably connected to the valve body proximate the distal port to secure proximal end of guide catheter 228 thereto.

In one embodiment hemostasis valve 402 includes a bleedback valve used to reduce the blood that may be lost during an interventional procedure. The bleedback valve acts to allow an elongated device such as a guide wire to extend therethrough but minimizes blood loss through the valve. In one embodiment hemostasis valve 402 includes a Tuohy-Borst adaptor that allows for the adjustment of the size of the opening in proximal end. Rotation of an engagement member about the valve's longitudinal axis acts to increase or decrease the diameter of the opening.

In one embodiment the bleedback valve is opened from a closed position to a fully opened position with a single motion or translation of an engagement member. In one example an engagement member is push or pulled along the longitudinal axis of the elongated medical device to fully open or fully close the valve. Some hemostasis valve devices include both type of controls, a rotational engagement member that opens and closes the tuohy borst valve by rotation of the engagement member about the longitudinal axis of the engagement member and a push pull control in which the engagement member is moved along the longitudinal axis to open and close the bleedback valve. Other type of control mechanisms are also known such as using a lever or ratchet to open and close the valve.

Referring to Figure 29 and Figure 30 hemostasis valve 402 includes an engagement member 416 that provides operation of the Tuohy-Borst valve by rotation of engagement member 416 and a push pull adjustment of the bleedback valve between a fully open and closed position by moving the engagement member 416 along the longitudinal axis of the hemostasis valve.

Control of the Tuohy-Borst and bleed back valves is accomplished robotically from a remote control station 14 by a first drive member 406 operatively connected to a first driven member 404 to rotate engagement member about the longitudinal axis. In one embodiment first drive member is a drive gear and the driven member 404 is a beveled gear secured to engagement member 416 and operatively connected to a drive gear. A second drive member 412 is operatively connected to the engagement member to translate the engagement member 416 along the longitudinal axis of the hemostasis valve. In one embodiment, second drive member is a lever that is robotically controlled via a motor that is controlled by the remote control station 14. Lever 412 operatively engages a collar slot 414 in the outer periphery of engagement member 16 such that movement of the lever 412 results in the translation of the engagement member 416 which as discussed above opens the bleedback valve between the closed and open positions.

In one embodiment a user may operate the first drive member 412 and the second drive member 412 to open and close the bleedback and Tuohy-Borst valves by providing instructions through a user input to rotate and/or translate the engagement member 416 about and/or along the longitudinal axis. In one embodiment, first drive member 412 and the second drive member 412 are automatically operated by a remote robotic control station 14 in response to a sensor that senses the blood flow and/or fictional forces required to move an elongated medical device either through the hemostasis valve and or a patients' vasculature. When the system detects that the force required to robotically rotate and or translate the elongated medical device the system reaches some predetermined value the processor would provide instructions to incrementally open and or close the opening in one or both of the valves. Monitoring of a patient's blood pressure and or whether blood is being lost through the valve would be used as factors in an algorithm to determine the appropriate adjustment to the opening in the valves.

Referring to Figure 27, robotic catheter system 210 operates proximate a patient bedside system 12 adjacent a patient bed 22. A remote work station 14 includes a controller 16, a user interface 18 and a display 20. An imaging system 24 may be any medical imaging system that may be used in conjunction with a catheter based medical procedure (e.g., non-digital x-ray, digital x-ray, CT, MRI, ultrasound, etc.). In one embodiment, imaging system 24 is a digital x-ray imaging device that is in communication with workstation 14. Imaging system 24 is configured to take x-ray images of the appropriate area of patient during a particular procedure. For example, imaging system 24 may be configured to take one or more x-ray images of the heart to diagnose a heart condition. Imaging system 24 may also be configured to take one or more x-ray images during a catheter based medical procedure (e.g., real-time images) to assist the user of workstation 14 to properly position a guide wire, guide catheter, and a working catheter such as a stent during a procedure. The image or images may be displayed on display 20 to allow the user to accurately position a distal tip of a guide wire or working catheter into proper position in a patient's vasculature.

Referring to Figure 28 flexible track 216 extends along the longitudinal axis 248 toward the patient. However, during a procedure, the patient may move resulting in the sheath clip pulling away or toward the patient. In one embodiment flexible track 216 assumes an arc shape between the distal end of cassette 222 and the patient. Guide catheter 228 positioned within the cavity defined by flexible track 216 assumes the same arc shape as flexible track 216. If a patient moves during a procedure the away from cassette 222 the arc 390 will flatten. Similarly, if the patient moves during the procedure toward the cassette 222 the arc 390 will be more pronounced. In both circumstances flexible track 216 prevents guide catheter 228 from buckling during a PCI procedure.

Referring to Figure 30 in one embodiment a sheath clip 420 is positively received within a distal end of cassette 222. The distal end of flexible track 216 is secured to a sheath clip 410 adjacent radially extending handle portion 428, sheath clip 420 includes a groove 430 having an opening 432. The distal end of flexible track 216 is located within the bottom of groove 430. In the install position shown in Figure 31 the longitudinal axis of sheath clip 420 is co-axial with longitudinal axis 248 of robotic mechanism 212. Top position the sheath clip 420 and flexible track 216 in an operation position a user pulls handle portion 428 and extends sheath clip 420 and attached flexible track 216 in a direction away from the robotic mechanism 212 and toward a patient. In one embodiment there is no need to rotate guide sheath 420 relative to cassette 222. A user simply pulls sheath clip 420 distally in a direction away from robotic mechanism 212.

Referring to Figure 32 and Figure 33, sheath clip 420 includes an introducer sheath connector 424 that releasably engages an introducer sheath 422. Introducer sheath connector includes at least a portion that rotatably coupled to sheath clip 420 proximate handle portion 428. Introducer sheath connector 424 includes an arm 436 that releasably engages an outer surface of introducer 422 to operatively couple the introducer sheath to sheath clip 420. Arm 436 in the engaged position illustrated in Figure 33 prevents introducer sheath 422 from moving from sheath clip 420 along the longitudinal axis toward or away from the patient. A tube extending from introducer sheath 422 is captured between sheath clip 420 and arm 436.

Referring to FIG 34 in one embodiment robotic catheter system 500 controls the movement of a catheter such as a microcatheter. Robotic catheter system 500 is similar to the catheter system 210 described above with a number of additional features described herein. Where the features of robotic catheter system 500 are similar to the features of catheter system 210 the same reference numerals will be used. Robotic mechanism 500 as described herein robotically rotates and linearly advances/retracts a catheter such as a microcatheter in the same manner it would guide catheter 228. In one embodiment a catheter 502 is used in a procedure in which the catheter 502 extends through a y-connector hemostasis valve 504 and into a second catheter 506. In one embodiment catheter 502 is a catheter having a lumen through which another elongated medical device extends through. A microcatheter is a thin wall, small diameter catheter used in vascular procedures such a minimally invasive applications as is known in the art. Microcatheters are used for navigating a network of arteries found within the vasculature of the body. Catheter is a general term that includes various types of devices including but not limited to a microcatheter, intermediate catheter, support catheter, aspiration catheter and sheath.

In one embodiment catheter 502 is a microcatheter secured to robotic mechanism 212. Flexible track 216 is placed over microcatheter 502 in a similar manner to guide catheter 228. Referring to FIG 8 in one embodiment microcatheter 502 has a diameter and flexible nature such that it buckles proximate to opening 274 of flexible track 216. Guide catheter 218 having a diameter greater than the diameter of microcatheter 502 does not buckle proximate to opening 274. A clip 508 is releasably secured to a portion of flexible track 218 closely adjacent to coupler 420. As described hereinabove coupler 420 releasably couples flexible track 216 to a proximal end of sheath 422. Referring to FIG 32 sheath 422 is an introducer sheath. Referring to FIG 34 a guide catheter 506 guides a microcatheter 502. Stated another way microcatheter 502 moves through a lumen of guide catheter 506.

Referring to FIG 34 and 35 in one embodiment a hemostasis valve 504 is positioned intermediate coupler 420 and guide catheter 506. An adaptor 510 is removably secured to hemostasis valve 504 and coupler 420 is releasably coupled to adaptor 510.

Referring to FIG 34 and FIG 35, adaptor 510 includes a center body portion 512 defining an opening 514, a distal end portion 516 defining a channel portion 518 and a coupler portion 520 at the proximal end portion 522. In one embodiment center body portion 512 includes a first member 511 and a second member 513 extending from the distal end portion and the proximal end portion. Coupler portion includes a tab 524. In one embodiment tab 524 is intermediate terminal end 526 of proximal end portion and body portion 512. Cavity 514 surrounds proximal end 528 of hemostasis valve 504 and channel 518 surrounds a body portion 530 of hemostasis valve 504. In one embodiment distal end portion 516 includes a first and second leg 534 and 536 that flexibly spread apart as adaptor 510 is placed forced over body portion 530. Legs 534 and 536 spring back toward one another once body portion 530 clears the free ends of legs 534 and 536 and is fully within channel 518. In this manner distal end portion 516 snap fits onto body portion 530 of the y-connector hemostasis valve 504. The term snap fit as used herein is an assembly method used to releasably attach flexible parts together by pushing the parts interlocking components together. In one embodiment the term snap fit refers to an assembly method used to non-releasably attach flexible parts together. Stated another way the width of body portion 530 is greater than the distance between the terminal portions 536 and 538 of legs 534and 536. The width of body portion 530 is less than the distance between in the intermediate portions 540 and 542 of legs 534 and 536. Accordingly, as the body portion 530 is being pressed between legs 534 and 536, the terminal portions 536 and 538 will be forced apart to a stressed position until body portion is fully within intermediate portions 540 and 542 at which point the stored spring energy in legs 534 and 536 will force the terminal edges toward one another until they are in their original non-stressed position. This is referred to herein as a snap fit. Adaptor 510 is removably attached to y-connector hemostasis valve 504 along a vector direction perpendicular to the longitudinal axis of the adaptor. In one embodiment adaptor distal end connector 516 is removed from y-connector hemostasis valve by pivoting the y-connector 504 relative to the adaptor in a non-colinear direction with the longitudinal axis of the adaptor. Stated another way the y-connector hemostasis valve is removed from the distal end connector by pivoting the longitudinal axis of the adaptor relative to the longitudinal axis of the y-connector hemostasis valve in a non-colinear direction. In one embodiment adaptor 510 is radially or side loadable onto the y-connector hemostasis valve.

Referring to FIG 32 and FIG 33 flexible track coupler 420 releasably secures coupler portion 520 of adaptor 510 in a similar manner to coupling to sheath 422 with tab 524 serving a similar function to the side port tubing extending from the proximal end of introducer sheath 422. Coupler 420 extends over the outer portion of proximal portion 522 and a portion of coupler 420 is then rotated such that tab 524 is prohibited from moving in a direction away from flexible track 216 by arm 436

In one embodiment hemostasis valve 504 is a COPILOT hemostatic valve sold by Abbott, however other hemostasis valves, y-connectors or other devices known in the art available now and in the future may also be used. A portion of microcatheter extends into a catheter such as a guide catheter that is removably coupled to the hemostasis valve. Adaptor 520 may be designed to engage a specific hemostasis valve, y-connector or introducer sheath or may include a variable engaging portion capable of being removably secured to a variety of hemostasis valve, y-connector or introducer sheath geometries. For example, a universal adaptor concept a portion of the adaptor either snap fits onto a portion of a y-connector leg or is mechanically fastened and/or clamped with a housing of the y-connector while still allowing rotation of outer surface (valve nut or locking nut or valve adjusting nut) of the hemostatic valve portion. Rotation of the valve nut adjusts the opening of the internal valve typically a Touhy-Borst valve. In one embodiment movement of the valve nut in the first direction (distally) fully opens the internal valve, while rotation about the first direction progressively adjusts the opening of the internal valve. In one embodiment a valve of the y-connector hemostasis valve 504 is opened with linear motion with respect to the body of the y-connector hemostasis valve. Linear motion in one embodiment is accomplished by applying a linear force to the proximal end of the y-connector hemostasis valve. In one embodiment the linear direction is parallel to the longitudinal axis of the y-connector hemostasis valve. Stated another way a valve of the y-connector hemostasis valve is opened by moving the outer member in a linear direction with respect to the body of the y-connector hemostasis valve within the opening of the body of the adaptor. In one embodiment when the y-connector hemostasis valve body is attached to the adaptor the valve is opened solely with linear motion in a linear direction along the longitudinal axis of the adaptor.

Referring to FIGS 37 clip 508 includes a handle portion 544 a grip portion 546 a proximal portion 552 and a beveled end 554 at the terminal end of the proximal portion 552. Grip portion 546 includes a plurality of arcuately shaped first pair of legs 548 and a second pair of legs 550. In one embodiment grip portion 546 includes a single pair of legs and in one embodiment grip portion includes more than two pair of legs. In one embodiment grip portion includes a plurality of leg portions that are offset from one another.

Clip 508 is removably coupled to an outer portion of flexible track 216 intermediate robotic drive 560 and coupler 420. An operator presses clip 508 such that grip portion 546 releasably grips an outer portion of flexible track 216. The outer diameter of flexible track 216 is greater than the distance between the terminal ends of each pair of legs such that the flexible track must deform to enter a channel region defined by the fingers. In one embodiment the inner diameter of the channel is greater than the outer diameter of the outer flexible track. In one embodiment inner diameter of channel is equal to or less than the outer diameter of flexible track 216. Clip 508 is then slid along flexible track in a direction away from robotic drive 560 toward coupler 420 until clip 508 covers opening 274. A portion 600 of clip 508 is received within a proximal portion of sheath clip 420.

Referring to FIG 40A clip 508 includes a beveled portion 554 at the free end of proximal portion 552. If robotic drive 560 moves toward coupler 420 such that a distal terminal end 563 of catheter drive 560 contacts clip 508 beveled portion 554 will contact terminal end 563 and automatically force clip 508 off of flexible track 216.

Referring to FIG 39 when clip safety portion 554 contacts distal portion 563 of cassette 222 or base 212 and a force greater than a release force is applied therebetween fingers 548 and fingers 550 of clip 508 will release from flexible track. The proximal end of Clip 508 moves in a direction away from the local longitudinal axis of the flexible track while the distal portion of clip 508 generally pivots within the proximal portion of sheath clip 420. In one embodiment flexible track 216 is releasably connected to sheath coupler 420 and will separate from sheath coupler 420 upon a disengagement force. The release force upon which the clip 508 disengages from flexible track 216 is less than the disengagement force of the flexible track 216 from sheath coupler 420. In this manner when the flexible track is withdrawn or moved in a proximal direction and clip 508 contacts the cassettes or base, clip 508 will be released from flexible track prior to flexible track 216 disengaging from sheath coupler 420.

In one embodiment coupler 420 and adaptor 510 are integrated into a single component. The use of adaptor 510 as a separate component allows flexible track 216 to be used for PCI in which coupler 420 to be connected directly to an introducer sheath and also be used for NVI where a microcatheter is used that require a hemostasis valve that is intermediate coupler 420 and an introducer sheath.

Hemostatic valve 504 includes a rotatable outer member 505 or nut that rotates about a longitudinal axis of hemostatic valve 504 to tighten and loosen a valve (not shown). Adaptor opening 514 has sufficient distance between first member 511 and second member 513 to allow a user to rotate the outer member 505 when the adaptor has been secured to body portion 530 of the hemostasis valve 504. In one embodiment outer member 505 may be rotated from one or both sides of opening 514. Where a first side is adjacent edge 515 and a second side is adjacent edge 517

Referring to FIG 40, 41 and 42 a cover 568 is pivotally secured to a portion of cassette 222 allowing a user to freely located a proximal hub of a guide catheter or microcatheter or other elongated medical device within the rotational drive mechanism. Cover 568 includes a first region providing sufficient clearance between the rotation drive mechanism and the underside 580 of cover 558. The elongated medical device 502 extends from rotational drive a distance 578 till the elongated medical device 502 is supported by flexible track 216. Cover 268 includes a second portion 572 having an underside portion that is adjacent to the elongated medical device 502 when cover 568 is in the closed position. In one embodiment second portion 572 has a tapered portion tapering from a first proximal portion 574 to a distal portion 576. The underside of second portion 572 has sufficient geometry to allow various elongated medical device of various diameters to extend rotate about its longitudinal axis when the cover is closed without buckling along the distance 578. Cover 568 supports the proximal end of the EMD such as a microcatheter (before it is supported by the flexible track or any other support feature). In one embodiment cover portions 570 and 572 can be two separate components that move independently of one another and/or are formed of two separate components. In one embodiment cover components 570 and 572 are formed as a unitary component such as a continuously unitary molded component. As such cover portion 572 supporting proximal end of the EMD (elongated medical device) can be decoupled from the features of portion 570 which hold the geared adaptor of the rotational drive in place and allow the EMD to rotate as well as to keep the Y-connector body portion from rotating.

Referring to FIG 43 a catheter system 500 is a triple coaxial system know in the art as a triaxial system including a catheter 506 having a hollow lumen that receives an intermediate catheter 582 therein. Intermediate catheter 582 has a hollow lumen that receives a controlled catheter 502 therein. The controlled catheter 502 is controlled by robotic mechanism to impart linear and rotary motion to controlled catheter 502. In one embodiment catheter 506 is a guide catheter and intermediate catheter 582 is a support catheter and controlled catheter 502 is a microcatheter having a hollow lumen that receives guidewire 584 therein. In one embodiment guide catheter 506 is a long sheath or guiding sheath. In contrast referring to FIG 34 a biaxial system includes a controlled catheter with a hemostasis valve and a guide catheter connected thereto. In one embodiment controlled catheter is one of a microcatheter and a support catheter.

A guidewire 584 is operatively controlled by robotic mechanism 212 and alone or with a catheter device such as a balloon or stent catheter (not shown in FIG 43) or other percutaneous devices extends through y-connector 233 (see FIG 2). Controlled catheter 502 is connected to the y-connector 233 at a proximal end of controlled catheter 502 and distal portion of y-connector 233. In one embodiment y-connector 233 includes a hemostasis valve, the combination of which is referred to herein as a y-connector hemostasis valve. Referring to FIG 43A a guidewire 584 is located within a hollow lumen of controlled catheter 502 that is located within flexible track 216. Flexible track 216 has a slit extending from the outer surface of track 216 to the inner surface of track 216 allowing the flexible track 216 to be placed over microcatheter 502. Controlled catheter 502 extends through an intermediate catheter y-connector hemostasis valve 504 and into intermediate catheter 582. Intermediate catheter 582 includes a proximal end with a connector that is operatively connected to the intermediate catheter y-connector 504. In one embodiment, intermediate catheter y-connector 504 includes a hemostasis valve. Intermediate catheter y-connector 504 is releasably connected to adaptor 510 which is operatively connected to flexible track 216 as described above. In one embodiment the adaptor distal end connector is removably connected to the y-connector hemostasis valve while the catheter is extending through the y-connector hemostasis valve. Guidewire 584 may also be, but not limited to, the pusher wire for a stent retriever, self-expanding stent, embolization coil, or flow divertor.

Referring to FIG 43B, guidewire 584 and controlled catheter 502 extend through a hollow lumen of intermediate catheter 582 and intermediate catheter y-connector hemostasis valve 504. Note that FIGS 43A - 43D are schematic and not to scale to illustrate the relative location and relative sizes of each of the devices.

A distal y-connector 586 which in one embodiment is a y-connector hemostasis valve is positioned distal the intermediate catheter y-connector hemostasis valve 504. The guidewire 584, controlled catheter 502 and intermediate catheter 582 extend through the distal y-connector hemostasis valve 586 and referring to FIG 43C extend into a hollow lumen of catheter 506 which in one embodiment is a guide catheter. Guide catheter 506 has a proximal end connector which is removably connected to the distal portion of distal y-connector 586.

Referring to FIG 43 and FIG 43D, guidewire 584, controlled catheter 502, intermediate catheter 582, and catheter 506 all are received within a hollow lumen of introducer sheath 422. In one embodiment, controlled catheter y-connector includes a hemostasis valve. In one embodiment intermediate y-connector includes a hemostasis valve. In one embodiment all of the y-connectors include a hemostasis valve. In one embodiment in any combination some but not all of the y-connectors include a hemostasis valve.

Referring to FIG 43E a general cross section taken along lines 43A-43A of FIG 43 provides an example of a catheter 585 such as a balloon catheter being located within a controlled catheter 503 along with guidewire all three of which are within flexible track 216.

In one embodiment one or more additional intermediate catheters and y-connectors are positioned between the intermediate y-connector 586 and introducer sheath 422. In one embodiment catheter 506 or guide catheter is attached to the last of the additional y-connectors prior to the various devices extending through the introducer sheath. In one embodiment intermediate hemostasis valve 586 is not attached to an adaptor 510. In one embodiment controlled catheter y-connector hemostasis valve 504 and intermediate y-connector hemostasis valve 586 are not supported by the cassette 222 or base 214. In one embodiment controlled catheter y-connector hemostasis valve 504 and intermediate y-connector hemostasis valve 586 are supported by the cassette 222, base, or other support fixed relative to patient and/or patient bed. In one embodiment a robotic catheter system includes a robotic drive having a first actuator manipulating a guidewire and a second actuator manipulating a controlled catheter. A support track extending from the robotic drive releasably receiving the controlled catheter. An adaptor releasably coupling a body portion of an intermediate catheter y-connector hemostasis valve, the adaptor has a proximal end connector operatively releasably coupling to the support track. An intermediate catheter having a proximal end connector releasably secured to a distal end connector of the intermediate catheter y-connector hemostasis valve, the controlled catheter extending within a hollow lumen of the intermediate catheter. Wherein in one embodiment the controlled catheter is a microcatheter 502 and the intermediate catheter is a guide catheter 506. Referring to FIG 43 in one embodiment controlled catheter is microcatheter 502 and the intermediate catheter is intermediate catheter 582.

As illustrated below a user may select a loading configuration through a user input that is shown on a display. A user input may be a joystick, mouse, touch screen, touch buttons or any other known input device that allows a user to select between more than one loading configuration option. Referring to FIG 44 an exemplary screen shot 700 allows a user to select between various operations of the catheter robotic system such as volume, system status and system configuration. Referring to FIG 45 when a user selects the system configuration button or graphical user interface on a touch screen representative of system configuration option the user will be presented with at least two options to choose for different loading positions. In one embodiment the options are presented as a dropdown menu 702 as a graphical user interface on a display such as a computer monitor. In one embodiment the first option of system configuration is a default option which may be preset by the manufacturer or may be set by a system administrator or a user. In one embodiment the default option is a center loading configuration and is not part of the drop down menu.

In one embodiment a first option is a "center loading zone" and a second option is a "rear loading zone". These two options determine a starting location of the robotic drive relative to the base. Referring to FIG 46 a screen graphic 704 displays a robotic mechanism 212 is in a center position such the robotic mechanism 212 can be moved both toward and away from the patient in equal distances. Robotic mechanism 212 is moved relative to base 214. The center position represents the position of the robotic mechanism relative to base 214 such that the robotic mechanism can be moved equal distances in both the direction toward the patient and in the opposite direction away from the patient. Stated another way the center position represents the position of the robotic mechanism relative to base 214 such that the robotic mechanism can be moved equal distances in a first direction along the longitudinal axis of the robotic mechanism and in a second direction opposite to the first direction from the center starting position. Referring to FIG 46 when a center or default loading zone is active the user may select to switch to a rear loading position by selecting a button 706 either via a touch screen or with a user input on the user screen as is known in the art.

Referring to FIG 46 in one embodiment the graphic display indicates the current location of the robotic mechanism 212 relative to the base214 with an indicator 708. In one embodiment indicator 708 is a green bar representing a target zone illustrating the general location of the robotic mechanism 212 for center loading and a vertical bar 710 (which in one embodiment is red) indicates the specific location of the robotic mechanism 212 within the target zone 708.

In one embodiment robotic mechanism 212 can move relative to base 214 a set distance in a first direction along a longitudinal axis of the robotic mechanism 212 toward a patient and a second direction opposite along the longitudinal axis of the robotic mechanism 212 away from the patient. By way of example if the total movement of robotic mechanism 212 from a rearward most position to a forward most position is 100 units a center loading zone would place the robotic mechanism 212 relative to base 214 such that from the center location the robotic mechanism 212 is movable relative to the base 214 from the center position 50 units in the first direction and 50 units in the second direction. When the robotic mechanism 212 is moved from the center position 50 units in the first direction the robotic mechanism would then be in the forward most position in this forward most position robotic mechanism cannot move any further in the first direction. Referring to FIG 47 a rear loading target position 709 identifies the target zone of the robotic mechanism 212 respect to the base 214 for rear loading. In one embodiment a vertical red bar will only be visible within the loading zone 709 when robotic mechanism 212 is within the predetermined rear loading zone. When in the rear loading zone configuration it is possible to switch to the center load position via a button 710.

When the robotic mechanism 212 is moved from the center position 50 units in the second direction the robotic mechanism 212 is in the rearward most position in this rearward most position robotic mechanism cannot move any further in the second direction.

In one embodiment a rearward position is intermediate the center position and the rearward most position. In the example noted above the robotic mechanism 212 can move 100 units from a rearward most position to a forward most position of 100 units of possible total travel. The units can be in inches, centimeters or some other definable unit. In one example where the user would like to have the option of moving a catheter device such as a microcatheter in the first direction up to 75 units, a user would select a rear loading position that is positioned 25 units in the second direction from the center position. In this rear loading position a user can move the microcatheter up to 25 units in the second direction from the rear loading position and 75 units in the first direction from the rear loading position.

A user may select a predetermined loading position from the drop down menu accessible from a touch screen monitor proximate the robotic mechanism that is positioned bed side along with the robotic mechanism and/or from an input device in a controller located distal from the robotic mechanism 212 in a cockpit protected with a radiation shield that is spaced from and not physically supported by one or more of the patient bed, the robotic mechanism 212 or the base 214.

Where a procedure requires an elongated medical device to be withdrawn such as an imaging device such an intravascular ultrasound device (IVUS), a user position the robotic mechanism in the forward most position when the EMD is the fully inserted position. In this manner a user may withdraw the EMD the full possible about of linear travel of the robotic mechanism. Note that the EMD such as a guide catheter is moved along the first direction (insert into a patient) and opposing second direction (withdrawing from a patient) by move the entire robotic mechanism 212 since the proximal end of the EMD is fixed relative to the cassette along the cassette and/or robotic mechanism longitudinal axis. In one embodiment in addition to the center and rear loading positions a forward loading position is also available in the drop down system configuration menu with a target bar in the forward most position. The available axial movement of the distal portion of the elongated medical device is a function of the loading position of the catheter mechanism.

Where an operator wants to drive a microcatheter or other catheter device into a vasculature or other region of a patient it is desirable to start the robotic mechanism 212 in a rearward position. In the rearward position the robotic mechanism can travel in the first direction along its longitudinal axis toward the patient a greater distance than the robotic mechanism can travel from the rearward position in the second direction away from the patient. In one embodiment in the rearward position the robotic mechanism 212 may move relative to the base at least 25 mm in the second direction. The second direction is in a direction away from the patient.

The ability to move the robotic mechanism 212 in the first or second direction a few units or fraction of a unit to allow for fine tuning the location of the proximal end or hub of the guide catheter or microcatheter or elongated device within the guide wire or microcatheter rotational drive in the cassette of the robotic mechanism 212.

In one embodiment when an operator selects the rear loading zone button, the robotic mechanism 212 automatically is moved to the rearward position. When an operator selects the center loading position the robotic mechanism 212 automatically is moved to the center position. Where a default loading zone has been selected either by the system or by a user the robotic mechanism 212 moves automatically to the location relative to base 214 when the default is selected.

Movement of robotic mechanism 212 relative to the base along a linear guide in the first direction and opposing second direction is controlled by a user input at the control and is also controlled by a user input attached to robotic mechanism 212. The user input at the control in one embodiment includes a joystick. Other input devices known in the art may also be used. The user input attached to robotic mechanism 212 includes a first button which when selected by a user moves the robotic mechanism in the first direction and a second button which when selected by a user moves the robotic mechanism 212 in the second direction. In one embodiment first button must be held down continuously by a user for the robotic mechanism to move in the first direction and second button must be held down continuously by a user for the robotic mechanism 212 to move in the second direction. Stated another way when a user contacts or holds down first button robotic mechanism 212 moves in the first direction and as soon as the user stops contact with or stops holding down first button robotic mechanism 212 ceases movement in the first direction.

In one embodiment robotic mechanism 212 includes a first button 712 and a second button 714 to move the robotic mechanism 212 in the first direction and a second direction respectively. In one embodiment the first direction is a distal direction and the second direction is a proximal direction as in known in the art. Fine adjust buttons 712 ,714 are used to move the robotic mechanism 212 to properly seat the proximal end of the guide catheter or microcatheter or other elongated medical device to property seat within the elongated medical device support and/or rotational drive mechanism of the robotic mechanism 212.

Referring to FIG 48 robotic mechanism 212 includes a cassette 222 that is operatively secured to robotic drive base 220. In one embodiment the longitudinal axis of cassette 222 is substantially perpendicular to the direction of gravity when cassette 222 is secured to drive base 220. This orientation will be referred to herein as the horizontal position. A location of cassette 222 and the operation of the various drive mechanism of the robotic mechanism 212 are automatically tested when cassette 222 is in the horizontal position. In one embodiment a user may select a loading position as described above and the robotic mechanism 212 will move automatically to the selected loading position. In one embodiment when a user selects a loading position, the user then must use the bedside buttons 712, 714 discussed above used for fine tuning to move the robotic mechanism 212 to the desired loading position. In this user movement option, a graphic appears on the bedside monitor or remote display monitor illustrating the position of the robotic mechanism relative to base. In one embodiment the loading position is a position in which robotic mechanism 212 is level with the base drive and an operating position is a position in which robotic mechanism 212 is directed toward the patient an angle relative to the drive base.

Robotic mechanism 212 can be positioned such that the longitudinal axis of the robotic mechanism 212 is not perpendicular to the direction of gravity. In one example robotic mechanism 212 rotates 30 degrees from the horizontal position. In one embodiment the robotic mechanism 212 is automatically locked into rotational angle of 30 degrees upon rotational movement of the robotic mechanism 212 from the horizontal relative to the base. Rotational movement of robotic mechanism 212 from the horizontal in a counter clockwise orientation such that the proximal end of the robotic mechanism is below portion of the longitudinal axis while the proximal end of the robotic mechanism is above the longitudinal axis of the robotic mechanism 212 when the robotic mechanism was in the horizontal position.

The 30 degree angled position of the robotic mechanism 212 allows the elongated medical device to be pointed toward the patient during a procedure. In one embodiment the angled position represents the operational position where an elongated medical device extends from the robotic mechanism 212 into the patient.

In one embodiment a linear actuator allows for limited travel of the robotic mechanism 212, the system can be setup to allow for movement in both the first direction and second direction or biased in one direction versus the other direction.

In one embodiment during a PCI procedure the distal tip of the guide catheter is manually engaged into the coronary artery ostium before the proximal end of the guide catheter is loaded in the system. Central loading position allows the user to advance the GC (guide catheter) to deep seat the distal tip of the GC or to reestablish engagement if the GC backed out of the coronary ostium or to retract to make sure it doesn't seat to deeply or to disengage the GC from the coronary ostium. In one embodiment the system doesn't need to be set up exactly in the center of the linear travel. It may be advantageous to allow for move retraction (second direction) or more advancement (in first direction) of the catheter.

In one embodiment the loading position is determined by data collection over time with various devices and patient anatomy to optimize the best loading position to, for instance, ensure that there will be enough advance travel to reestablish coronary ostium engagement in a patient with an enlarged ascending aorta or to ensure that the device will be able to advance to the target such as lesion or beyond a target to support delivery of therapy.

In one embodiment for microcatheter (or support catheter) use, the distal tip is inserted into a catheter (guide catheter, sheath or intermediary catheter) just prior to the tip of the catheter, then it is loaded in the system. In this case, the user will advance the microcatheter or elongated medical device (EMD) to a target. However, since all the devices are compliant, in one embodiment the system allows for some retraction from the loading position. Stated another way the system may not be biased all the way to one end. The rear loading position in one embodiment sets up for 175 mm advancement and 25 mm retraction. This also allows for fine adjustment of the robotic drive position to load the EMD once the positioning system has already been setup.

While the foregoing written description of the invention enables one of ordinary skill to make and use what is considered presently to be the best mode thereof, those of ordinary skill will understand and appreciate the existence of variations, combinations, and equivalents of the specific embodiment, method, and examples herein. The invention should therefore not be limited by the above described embodiment, method, and examples, but by all embodiments within the scope of the invention as claimed.

## Claims

1. A robotic catheter (500) system comprising:
a robotic drive (560) having a first actuator manipulating a guidewire (584) and a second actuator manipulating a controlled catheter (502);
a support track extending from the robotic drive (560) releasably receiving the controlled catheter (502);
an adaptor (510) releasably coupling a body portion (530) of an intermediate catheter y-connector hemostasis valve (504), the adaptor (510) has a proximal end connector (520) operatively releasably coupling to the support track (216);
the adaptor (510) including:
a distal end connector (516) configured to engage a body portion (530) of the hemostasis valve (504);
a proximal end connector (520) configured to operatively connect to an elongated medical device support track (216);
wherein the adaptor (510) includes a body (512) defining an opening (514) configured to encompass an outer member (505) of the hemostasis valve (504), the outer member (505) being rotatable within the opening (514) about a longitudinal axis of the hemostatic valve (504) to tighten and loosen the hemostasis valve (504); and
the robotic catheter (500) system comprising an intermediate catheter (582) having a proximal end connector releasably secured to the distal end connector (516) of the intermediate catheter y-connector hemostasis valve (504), the controlled catheter (502) extending within a hollow lumen of the intermediate catheter (582).

2. The robotic catheter system (500) of claim 1, further including a distal y-connector hemostasis valve (586) through which the intermediate catheter (582) extends and a guide catheter (506) having a proximal end connector releasably connected to the distal end of the distal y-connector hemostasis valve (586), the guide catheter (506) having a hollow lumen through which the controlled catheter (502) and the intermediate catheter (582) extend.

3. The robotic catheter system (500) of claim 1, wherein the controlled catheter (502) is one of a microcatheter and a support catheter.

4. The robotic catheter system (500) of claim 3, wherein the adaptor (510) is releasably coupled to the intermediate catheter y-connector hemostasis valve (504) while the controlled catheter (502) extends through and exits the intermediate catheter y-connector hemostasis valve (504).

5. The robotic catheter system of claim 1, wherein the adaptor (510) has a longitudinal axis that is co-axial with a longitudinal axis of the intermediate catheter y-connector hemostasis valve (504).

6. The robotic catheter system of claim 1, wherein the opening (514) in the body (512) is defined by a first arm (511) and a second arm (513) extending intermediate the distal end connector (516) and the proximal end connector (520) of the adaptor (510).

7. The robotic catheter system of claim 1, wherein the intermediate catheter y-connector hemostasis valve (504) and the distal end connector (516) is removably connected to the portion (530) of the intermediate catheter y-connector hemostasis valve (504) which is non non-rotating.

8. The robotic catheter system of claim 7, wherein the distal end connector (516) is connected to the portion (530) of the intermediate catheter y-connector hemostasis (504) with a snap fit.

9. The robotic catheter system of claim 8, wherein the intermediate catheter y-connector hemostasis valve (504) is removed from the distal end connector (516) by pivoting the longitudinal axis of the adaptor (510) relative to the longitudinal axis of the intermediate catheter y-connector hemostasis valve (504) in a non-colinear direction.

10. The robotic catheter system of claim 8, wherein the adaptor (510) is radially connected to the portion (530) of the intermediate catheter y-connector hemostasis valve (504) in a direction perpendicular to the longitudinal axis of the intermediate catheter y-connector hemostasis valve (504).

11. The robotic catheter system of claim 8, wherein a valve of the intermediate catheter y-connector hemostasis valve (504) is opened by moving the outer member (505) solely in a linear direction with respect to the body (530) of the controlled y-connector hemostasis valve (504) within the opening (514) of the body (512) of the adaptor (510).

12. The robotic catheter system of claim 1, wherein the support track includes a flexible tube having a slit (286) extending substantially the entire length of the tube, the support track (216) including a distal end (254) with a coupler at the distal end (254) that connects to the proximal end connector (520) of the adaptor (510).

13. The robotic catheter system (500) of claim 1, wherein a valve of the y-connector hemostasis valve (504) is opened by moving the outer member (505) in a linear direction with respect to the body (530) of the y-connector hemostasis valve (504) within the opening (514) of the body (512) of the adaptor (510).

## Patentansprüche

1. Robotisches Kathetersystem (500) umfassend:
einen robotischen Antrieb (560) mit einem ersten Aktuator, der einen Führungsdraht (584) manipuliert, und einem zweiten Aktuator, der einen gesteuerten Katheter (502) manipuliert;
eine Trägerbahn, die sich von dem robotischen Antrieb (560) erstreckt und den gesteuerten Katheter (502) lösbar aufnimmt;
einen Adapter (510), der einen Körperabschnitt (530) eines Zwischenkatheter-Y-Verbinder-Hämostaseventils (504) lösbar koppelt, wobei der Adapter (510) einen proximalen Endverbinder (520) zur lösbaren Wirkkopplung mit der Trägerbahn (216) aufweist;
wobei der Adapter (510) umfasst:
einen distalen Endverbinder (516), ausgelegt zum Eingriff mit einem Körperabschnitt (530) des Hämostaseventils (504);
einen proximalen Endverbinder (520), ausgelegt zur Wirkverbindung mit einer langgestreckten Trägerbahn (216) der medizinischen Vorrichtung;
wobei der Adapter (510) einen Körper (512) aufweist, der eine Öffnung (514) definiert, die dazu ausgelegt ist, ein äußeres Element (505) des Hämostaseventils (504) zu umgeben, wobei das äußere Element (505) innerhalb der Öffnung (514) um eine Längsachse des Hämostaseventils (504) drehbar ist, um das Hämostaseventil (504) festzuziehen und zu lösen; und
wobei das robotische Kathetersystem (500) einen Zwischenkatheter (582) mit einem proximalen Endverbinder umfasst, der lösbar an dem distalen Endverbinder (516) des Zwischenkatheter-Y-Verbinder-Hämostaseventils (504) befestigt ist, wobei der gesteuerte Katheter (502) innerhalb eines hohlen Lumens des Zwischenkatheters (582) verläuft.

2. Robotisches Kathetersystem (500) nach Anspruch 1, ferner umfassend ein distales y-Verbinder-Hämostaseventil (586), durch das der Zwischenkatheter (582) verläuft, und einen Führungskatheter (506) mit einem proximalen Endverbinder, der lösbar mit dem distalen Ende des distalen y-Verbinder-Hämostaseventils (586) verbunden ist, wobei der Führungskatheter (506) ein hohles Lumen aufweist, durch das der gesteuerte Katheter (502) und der Zwischenkatheter (582) verlaufen.

3. Robotisches Kathetersystem (500) nach Anspruch 1, wobei der gesteuerte Katheter (502) eines von einem Mikrokatheter und einem Trägerkatheter ist.

4. Robotisches Kathetersystem (500) nach Anspruch 3, wobei der Adapter (510) lösbar an das Zwischenkatheter-y-Verbinder-Hämostaseventil (504) gekoppelt ist, während der gesteuerte Katheter (502) durch das Zwischenkatheter-y-Verbinder-Hämostaseventil (504) verläuft und daraus austritt.

5. Robotisches Kathetersystem nach Anspruch 1, wobei der Adapter (510) eine Längsachse aufweist, die koaxial mit einer Längsachse des Zwischenkatheter-y-Verbinder-Hämostaseventils (504) ist.

6. Robotisches Kathetersystem nach Anspruch 1, wobei die Öffnung (514) in dem Körper (512) durch einen ersten Arm (511) und einen zweiten Arm (513) definiert ist, die zwischen dem distalen Endverbinder (516) und dem proximalen Endverbinder (520) des Adapters (510) verlaufen.

7. Robotisches Kathetersystem nach Anspruch 1, wobei das Zwischenkatheter-y-Verbinder-Hämostaseventil (504) und der distale Endverbinder (516) entfernbar mit dem Abschnitt (530) des Zwischenkatheter-y-Verbinder-Hämostaseventils (504), der sich nicht dreht, verbunden sind.

8. Robotisches Kathetersystem nach Anspruch 7, wobei der distale Endverbinder (516) über eine Schnappverbindung mit dem Abschnitt (530) des Zwischenkatheter-y-Verbinder-Hämostaseventils (504) verbunden ist.

9. Robotisches Kathetersystem nach Anspruch 8, wobei das Zwischenkatheter-y-Verbinder-Hämostaseventil (504) von dem distalen Endverbinder (516) entfernt wird, indem die Längsachse des Adapters (510) relativ zu der Längsachse des Zwischenkatheter-y-Verbinder-Hämostaseventils (504) in einer nicht-kolinearen Richtung geschwenkt wird.

10. Robotisches Kathetersystem nach Anspruch 8, wobei der Adapter (510) radial mit dem Abschnitt (530) des Zwischenkatheter-y-Verbinder-Hämostaseventils (504) in einer Richtung senkrecht zu der Längsachse des Zwischenkatheter-y-Verbinder-Hämostaseventils (504) verbunden ist.

11. Robotisches Kathetersystem nach Anspruch 8, wobei ein Ventil des Zwischenkatheter-y-Verbinder-Hämostaseventils (504) geöffnet wird, indem das äußere Element (505) ausschließlich in einer linearen Richtung bezogen auf den Körper (530) des gesteuerten y-Verbinder-Hämostaseventils (504) innerhalb der Öffnung (514) des Körpers (512) des Adapters (510) bewegt wird.

12. Robotisches Kathetersystem nach Anspruch 1, wobei die Trägerbahn einen flexiblen Schlauch mit einem Schlitz (286) aufweist, der im Wesentlichen über die gesamte Länge des Schlauchs verläuft, wobei die Trägerbahn (216) ein distales Ende (254) mit einem Koppler an dem distalen Ende (254) zur Verbindung mit dem proximalen Endverbinder (520) des Adapters (510) aufweist.

13. Robotisches Kathetersystem (500) nach Anspruch 1, wobei ein Ventil des y-Verbinder-Hämostaseventils (504) durch Bewegen des äußeren Elements (505) in einer linearen Richtung bezogen auf den Körper (530) des y-Verbinder-Hämostaseventils (504) innerhalb der Öffnung (514) des Körpers (512) des Adapters (510) geöffnet wird.

## Revendications

1. Système de cathéter robotisé (500), comprenant :
un mécanisme d'entraînement robotisé (560) comportant un premier actionneur manipulant un fil-guide (584) et un second actionneur manipulant un cathéter commandé (502) ;
une voie de support s'étendant à partir du mécanisme d'entraînement robotisé (560), recevant de manière libérable le cathéter commandé (502) ;
un adaptateur (510) s'accouplant de manière libérable à une partie corps (530) d'une valve hémostatique de cathéter intermédiaire à raccord en y (504), l'adaptateur (510) ayant un raccord d'extrémité proximale (520) s'accouplant fonctionnellement de manière libérable à la voie de support (216) ;
l'adaptateur (510) comportant :
un raccord d'extrémité distale (516) conçu pour entrer en prise avec une partie corps (530) de la valve hémostatique (504) ;
un raccord d'extrémité proximale (520) conçu pour se raccorder fonctionnellement à une voie de support de dispositif médical allongée (216) ;
dans lequel l'adaptateur (510) comprend un corps (512) définissant une ouverture (514) conçue pour recevoir un élément externe (505) de la valve hémostatique (504), l'élément externe (505) pouvant tourner dans l'ouverture (514) autour d'un axe longitudinal de la valve hémostatique (504) pour serrer et desserrer la valve hémostatique (504) ; et
le système de cathéter robotisé (500) comprenant un cathéter intermédiaire (582) ayant un raccord d'extrémité proximale fixé de manière libérable au raccord d'extrémité distale (516) de la valve hémostatique de cathéter intermédiaire à raccord en y (504), le cathéter commandé (502) s'étendant à l'intérieur d'une lumière creuse du cathéter intermédiaire (582).

2. Système de cathéter robotisé (500) selon la revendication 1, comprenant en outre une valve hémostatique distale à raccord en y (586) à travers laquelle s'étend le cathéter intermédiaire (582) et un cathéter de guidage (506) ayant un raccord d'extrémité proximale raccordé de manière libérable à l'extrémité distale de la valve hémostatique distale à raccord en y (586), le cathéter de guidage (506) ayant une lumière creuse à travers laquelle s'étendent le cathéter commandé (502) et le cathéter intermédiaire (582).

3. Système de cathéter robotisé (500) selon la revendication 1, dans lequel le cathéter commandé (502) est l'un d'un microcathéter et d'un cathéter de support.

4. Système de cathéter robotisé (500) selon la revendication 3, dans lequel l'adaptateur (510) est accouplé de manière libérable à la valve hémostatique de cathéter intermédiaire à raccord en y (504) tandis que le cathéter commandé (502) s'étend à travers et sort de la valve hémostatique de cathéter intermédiaire à raccord en y (504).

5. Système de cathéter robotisé selon la revendication 1, dans lequel l'adaptateur (510) a un axe longitudinal qui est coaxial à un axe longitudinal de la valve hémostatique de cathéter intermédiaire à raccord en y (504).

6. Système de cathéter robotisé selon la revendication 1, dans lequel l'ouverture (514) dans le corps (512) est définie par un premier bras (511) et un second bras (513) s'étendant entre le raccord d'extrémité distale (516) et le raccord d'extrémité proximale (520) de l'adaptateur (510).

7. Système de cathéter robotisé selon la revendication 1, dans lequel la valve hémostatique de cathéter intermédiaire à raccord en y (504) et le raccord d'extrémité distale (516) est raccordé de manière amovible à la partie (530) de la valve hémostatique de cathéter intermédiaire à raccord en y (504) qui n'est pas rotative.

8. Système de cathéter robotisé selon la revendication 7, dans lequel le raccord d'extrémité distale (516) est raccordé à la partie (530) de la valve hémostatique de cathéter intermédiaire à raccord en y (504) par encliquetage.

9. Système de cathéter robotisé selon la revendication 8, dans lequel la valve hémostatique de cathéter intermédiaire à raccord en y (504) est retirée du raccord d'extrémité distale (516) en faisant pivoter l'axe longitudinal de l'adaptateur (510) par rapport à l'axe longitudinal de la valve hémostatique de cathéter intermédiaire à raccord en y (504) dans une direction non colinéaire.

10. Système de cathéter robotisé selon la revendication 8, dans lequel l'adaptateur (510) est raccordé radialement à la partie (530) de la valve hémostatique de cathéter intermédiaire à raccord en y (504) dans une direction perpendiculaire à l'axe longitudinal de la valve hémostatique de cathéter intermédiaire à raccord en y (504).

11. Système de cathéter robotisé selon la revendication 8, dans lequel une valve de la valve hémostatique de cathéter intermédiaire à raccord en y (504) est ouverte en déplaçant l'élément externe (505) uniquement dans une direction linéaire par rapport au corps (530) de la valve hémostatique à raccord en y commandée (504) à l'intérieur de l'ouverture (514) du corps (512) de l'adaptateur (510).

12. Système de cathéter robotisé selon la revendication 1, dans lequel la voie de support comprend un tube flexible ayant une fente (286) s'étendant sensiblement sur toute la longueur du tube, la voie de support (216) comprenant une extrémité distale (254) avec un dispositif d'accouplement au niveau de l'extrémité distale (254) qui se raccorde au raccord d'extrémité proximale (520) de l'adaptateur (510).

13. Système de cathéter robotisé (500) selon la revendication 1, dans lequel une valve de la valve hémostatique à raccord en y (504) est ouverte en déplaçant l'élément externe (505) dans une direction linéaire par rapport au corps (530) de la valve hémostatique à raccord en y (504) à l'intérieur de l'ouverture (514) du corps (512) de l'adaptateur (510).
